# EUROPEAN PATENT SPECIFICATION

(11) **EP 4 362 773 B1**
(45) Date of publication and mention of the grant of the patent: **02.09.2026**
(21) Application number: 22741489.3
(22) Date of filing: 01.07.2022
(51) Int. Cl.: A61B 5/00

(54) **A FLEXIBLE AND STRETCHABLE COVER FOR ATTACHING A COMPONENT TO A PATCH**
FLEXIBLE UND DEHNBARE ABDECKUNG ZUR BEFESTIGUNG EINER KOMPONENTE AN EINEM PATCH
COUVERCLE FLEXIBLE ET EXTENSIBLE POUR FIXER UN COMPOSANT SUR UN TIMBRE

(30) Priority: 01.07.2021 EP 21183306
(43) Date of publication of application: 08.05.2024
(73) Proprietor: Datwyler Pharma Packaging Belgium NV, 3570 Alken (BE)
(72) Inventor: LAMBRECHTS, Tom, 3290 Diest (BE); BEHFAR, Mohammadhossein, 01200 Vantaa (FI); OTT, Roland, 74389 Cleebronn (DE)
(74) Representative: De Clercq & Partners
(86) International application number: PCT/EP2022/068225
(87) International publication number: WO 2023/275342

(56) References cited:
- WO-A1-2009/036313
- WO-A1-2011/081891
- WO-A1-2014/116816
- CN-A- 108 871 609
- US-A1- 2019 117 256

## Description

### FIELD

The invention relates to the technical field of wearable patches, more particularly to patches for attachment to the skin of a subject, for example in medical, lifestyle, wellness or sports applications, etc. Specifically, it relates to the field of patch assemblies comprising components, such as for example sensors, electronics circuits, processors, data communication modules, batteries, etc. and/or any other suitable electronic components, also referred to as smart patches.

### BACKGROUND

There is known an adherent patch device from WO2009/036313A1 for monitoring impedance and electrocardiogram signals. Such adherent patch devices are for example applied for physiological monitoring of patients. Especially when monitoring of a patient during longer periods of time is desired, the ability of wearing such a patch device by the patient in a comfortable way is desired.

WO2009/036313A1 discloses a cover which can be attached to an adherent patch, such that the cover stretches and/or retracts when the adherent patch stretches and/or retracts with the skin of the patient. Between the stretchable material of the adherent patch and the stretchable material of cover there are disposed components which do not stretch substantially, if at all, such as for example electronics components, a printed circuit board, an electronics housing. As described in WO2009/036313A1, these components are mounted on top of the electrodes which might limit the stretching and/or retracting properties of the cover and/or adherent patch.

Even when, according to embodiments described in WO2009/036313A1, the cover and the adherent patch are slidable with respect to those components, the mounting of these components, of which the position is determined by the position of the electrodes which are mounted fixedly and at fixed positions to the subject and which typically comprise a high rigidity, limits flexibility and stretchability of the patch device. According to such embodiments, similar as described in WO2009/036313A1, it is clear that this also provides the disadvantage of concentrating the majority of retaining forces with respect to these components at these specific mounting positions which are determined by the electrodes. With embodiments such as described in WO2009/036313A1, in this way, these retaining forces interact with the subject's skin in a concentrated way at these particular mounting locations, where the electrodes are attached to the skin.

A further disadvantage of embodiments such as described in WO2009/036313A1, as the electrodes themselves are typically less flexible or more rigid structures than the adherent patch, is that the retaining forces are thus transferred to the subject's skin at a location which is not able to adapt to any changes of the skin. It is clear, that a further disadvantage of embodiments such as described in WO2009/036313A1 relates to the transfer of the retaining forces for these components at the specific location of the electrodes, all retaining forces are thus concentrated at this limited contact surface, which also increases the risk of detachment and/or changes in the level of attachment of the electrodes, when subjected to changing retaining forces during use of the patch device. It is further clear, that in such prior art embodiments this leads to the disadvantage, that such changes could cause the risk of detachment of the electrodes or the risk that measurements by these electrodes are influenced by any of these changes.

It is thus clear that, in such prior art embodiments, this also will negatively affect the comfort of the subject, as the retaining forces for these components, which are typically the heaviest components of the patch device, are concentrated in these specific locations and will thus for example exert a stress on the skin of the patient in a concentrated way at these locations. In order to reduce these problems, WO2009/036313A1 proposes to adhere the upper surface of the electronics housing to the cover with an adhesive, in order to suspend the printed circuit module above the adherent patch by this connection to the cover. However, it is clear, that, in this prior art embodiment, such a connection to the cover of these components by means of adhesive, reduces the flexibility of the cover itself.

Further, according to such a prior art embodiment, in order to support the weight of these components, the cover needs to be provided with a sufficient level of stiffness, as otherwise the cover would collapse under the weight of these components, thereby not being able to guarantee the desired suspension. It is clear, that this increased level of stiffness of the cover would reduce the desired level of stretchability and flexibility of the cover. Further still, in events, where for example the patch device experiences shocks or sudden movements, for example resulting from a sudden movement or shock created by the subject itself during wear of the patch device, or caused by external events causing a sudden movement or shock to the patch device, according to such prior art embodiments, the desired gap between the PCB module and the adherent patch cannot be guaranteed. This is for example the case when the subject is performing physical action, for example during sports, physical exercise, physiotherapy, etc. , where monitoring of by means of such a wearable patch device is especially useful. It is clear, that all of the above disadvantages of the prior art embodiments, negatively affect patient comfort, robustness and safety of the patch device.

There is further known a patch device from WO2011/081891A1, which describes the use of bridging loops. These bridging loops are affixed to the adherent patch and to a circuit carrier and form a structure that controls motion of the circuit carrier with respect to the adherent patch permitting relatively free rotation of the circuit carrier about the X and Y axes illustrated in Figure 4 of WO2011/081891A1, and constraining the rotation of the circuit carrier about the Z axis. However, it is clear, that also in this prior art embodiment, especially when for example subjected to shocks, or sudden motions, especially in a direction directed away from and/or towards the adherent patch, that the desired gap between the PCB module and the adherent patch cannot be guaranteed. It is further clear that also according to this prior art embodiment, the retaining forces for retaining the components of the patch device are concentrated at the location of attachment of the bridging loops to the patch. It is thus clear, that all the above negatively affects patient comfort, robustness, and safety of the patch device.

Further the use of adhesive, the need for a connection at specific locations, and/or the use of additional mounting elements such as for example the bridging loops increases the complexity which reduces the efficiency with which the patch device can be manufactured and/or assembled. As, in the context of such patch devices, a quick, easy, and robust mounting and dismounting of components, such as batteries, electronics modules, etc. is frequently desired, these components limit robustness of the patch device as they are prone to failure and reduce efficiency of such mounting and dismounting operations.

Therefor there is a need for an improved patch assembly and/or parts thereof, which allow for a more robust mounting of the components while still allowing for an improved level of comfort, by for example an improved stretchability, flexibility and/or compliance with skin movement of the subject when components with a higher rigidity, reduced flexibility and/or reduced stretchability are mounted on such a patch.

### SUMMARY

The present invention is directed to a a patch for removeable attachment to the skin having a flexible and stretchable cover as defined in independent claim 1 as well as to the use of such a patch as defined in claim 13. Further aspects of the invention are defined in dependent claims.

To provide a solution to the above-described problems in the art, the present disclosure describes a flexible and stretchable cover and/or cover assembly configured for attachment to a patch for attachment to the skin of a subject, for example in medical, lifestyle, wellness, or sports applications, etc. The present cover defines a holding space for one or more components, such as for example sensors, processors, data communication modules, batteries, electronic connections, etc. and/or any other suitable electronic components, with the aim of attaching said components to the patch. Accordingly, the present disclosure also describes a patch assembly comprising cover and components, also referred to as a smart patch.

An aspect provides a flexible and stretchable cover configured for attachment to a patch and at least partially enclosing a component, whereby the cover comprises at least one flexible and stretchable clamping part which is configured:
- to clamp the component when mounted inside the cover in such a way that the clamping part is in contact with the component; and
- to apply a compression force to the component, thereby attaching the component to the inside of the cover.

In some preferred embodiments the clamping part is configured to clamp the component when mounted inside the cover in such a way that the clamping part is in slidable contact with the component.

In some preferred embodiments the cover is configured for attachment at one or more attachment points to a patch, wherein the patch is configured for attachment to skin of a subject.

In some preferred embodiments the cover is configured as a separate and/or different element from the patch.

In some preferred embodiments the cover is not configured for attachment to skin of the subject.

In some preferred embodiments only the cover is configured to clamp the component when mounted inside the cover.

In some preferred embodiments only the cover is configured to apply the compression force to the component.

In some preferred embodiments the patch is configured not to clamp the component when mounted inside the cover.

In some preferred embodiments the patch is configured to apply the compression force to the component when mounted inside the cover;
In some preferred embodiments the patch is configured not to contact the component when mounted inside the cover.

In some preferred embodiments the at least one clamping part is configured to apply the compression force to the component along at least one direction.

In some preferred embodiments the at least one clamping part is configured to apply the compression force to the component along a plurality of directions.

In some preferred embodiments the at least one clamping part is configured to apply the compression force inwards along at least a part of an outer surface of the component.

In some preferred embodiments the cover consists of a single-piece element.

In some preferred embodiments the cover comprises at least two flexible and stretchable clamping parts.

In some preferred embodiments the cover comprises at least two flexible and stretchable clamping parts arranged at opposing sides of the component when mounted.

In some preferred embodiments the cover comprises at least two flexible and stretchable clamping parts configured to apply the compression force to the component when mounted in between the at least two clamping parts.

In some preferred embodiments the cover comprises at least two flexible and stretchable clamping parts at opposing sides of the component when mounted, wherein the at least two flexible and stretchable clamping parts are arranged in such a way that the at least two flexible and stretchable clamping parts cooperate to apply the compression force to attach the component to the inside of the cover between the at least two flexible and stretchable clamping parts.

In some preferred embodiments the cover comprises, preferably consists, of a material, defined by a hardness in the range of at least 10 shore A to at most 90 shore A; preferably 20 shore A to 80 shore A; more preferably 30 shore A to 70 shore A; even more preferably 30 shore A to 60 shore A.

In some preferred embodiments the cover comprises, preferably consists, of a material, defined by a tensile strength in the range of at least 5.0 N/mm² to at most 11.0 N/mm²; preferably 5.5 N/mm² to at most 10.5 N/mm²; more preferably 6.0 N/mm² to at most 10.0 N/mm²; even more preferably 6.5 N/mm² to at most 9.5 N/mm²; even more preferably 7.0 N/mm² to at most 9.0 N/mm²; even more preferably 7.5 N/mm² to at most 8.5 N/mm².

In some preferred embodiments the cover comprises, preferably consists, of a material, defined by a maximal elongation in the range of at least 100% to at most 800%; preferably 200% to 800%; more preferably 300% to 750%; even more preferably 350% to 750%; even more preferably 400% to 700%; even more preferably 450% to 650%; even more preferably 500% to 600%.

In some preferred embodiments the cover comprises, preferably consists of a material, defined by a tear strength in the range of at least 20 to at most 30 N/mm; preferably 21 to 29 N/mm; more preferably 21 to 28 N/mm; even more preferably 22 to 27 N/mm; even more preferably 23 to 26 N/mm.

In some preferred embodiments the cover material comprises, preferably consists of, silicone rubber (SR), more preferably liquid silicone rubber (LSR); a thermoplastic elastomer (TPE), more preferably thermoplastic polyurethane (TPU), thermoplastic olefin (TPO), thermoplastic polyamide (TPA); a thermoplastic vulcanizate (TPV), a cross-linkable elastomer, more preferably isoprene rubber (IR), nitrile rubber (NBR), styrene-butadiene rubber (SBR), Ethlylene Propylene Diene Monomer (EPDM), fluorocarbon-based fluoroelastomer materials (FKM), polybutadiene rubber (BR); and/or combinations thereof.

A further aspect provides a cover assembly configured for attachment to a patch comprising the cover as described in the present disclosure, wherein the cover assembly further comprises the component mounted inside the cover by means of the at least one flexible and stretchable clamping part.

In some preferred embodiments the cover, preferably at least one flexible and stretchable clamping part of said cover, is in direct contact with the component over at least part of its interior surface; i.e. at least part of the interior surface of cover in direct contact with the exterior surface of component. In some preferred embodiments the cover, preferably at least one flexible and stretchable clamping part of said cover is provided with one or more inward projections that are in direct contact with the component, wherein optionally the inward projections are dimensioned to increase stretchability and/or flexibility at the location of direct contact with the clamping part. Preferably the one or more inward projections comprise a projection on the interior surface of the distal portion of the cover, a projection on the interior surface of the proximal portion of the cover, and/or a projection on the interior surface of the lateral portion of the cover.

In some preferred embodiments the at least one flexible and stretchable clamping part is configured such that, when subjected to changes resulting from flexing and/or stretching of the cover, the material of the clamping part in contact with the component is slidable with respect to the component. A further aspect provides a patch assembly configured for attachment to a subject's skin comprising:
- at least one cover assembly as described in the present disclosure, and
- a patch configured for removeable attachment to the skin of said subject, whereby the at least one cover assembly is attached to the patch by means of a cover of said cover assembly.

In some preferred embodiments the patch comprises, preferably consists of, a material, defined by a hardness in the range of at least 10 shore A to at most 90 shore A; preferably 20 shore A to 80 shore A; more preferably 30 shore A to 70 shore A; even more preferably 30 shore A to 60 shore A.

In some preferred embodiments the patch comprises, preferably consists of, a material, defined by a tensile strength in the range of at least 5.0 N/mm² to at most 11.0 N/mm²; preferably 5.5 N/mm² to at most 10.5 N/mm²; more preferably 6.0 N/mm² to at most 10.0 N/mm²; even more preferably 6.5 N/mm² to at most 9.5 N/mm²; even more preferably 7.0 N/mm² to at most 9.0 N/mm²; even more preferably 7.5 N/mm² to at most 8.5 N/mm².

In some preferred embodiments the patch comprises, preferably consists of, a material, defined by a maximal elongation in the range of at least 100% to at most 800%; preferably 200% to 800%; more preferably 300% to 750%; even more preferably 350% to 750%; even more preferably 400% to 700%; even more preferably 450% to 650%; even more preferably 500% to 600%.

In some preferred embodiments the patch comprises, preferably consists of, a material, defined by a tear strength in the range of at least 20 to at most 30 N/mm; preferably 21 to 29 N/mm; more preferably 21 to 28 N/mm; even more preferably 22 to 27 N/mm; even more preferably 23 to 26 N/mm.

According to some preferred embodiments, the component comprises and/or consists of one or more of the following:
- an electronic component;
- a non-electronic component;
- a reservoir;
- a dispenser.

According to some preferred embodiments, the component comprises a reservoir and/or dispenser configured to store and/or dispense of one or more of the following:
- a fluid;
- a spray;
- a solid;
- a medicament;
- cosmetics.

In some preferred embodiments the patch assembly further comprises:
- at least one sensor configured for measurement of a physiological status in electronic communication with the component, wherein the component comprises at least one electronic circuit configured to process, store and/or communicate the measurement of the sensor.

In some preferred embodiments the patch assembly further comprises a membrane which is permeable to gas and preferably impermeable to fluid.

According to some preferred embodiments, there is provided a patch assembly, wherein the cover comprises a proximal or an inferior portion configured to at least partially cover the side of the component which closest to the patch of the patch assembly, and wherein the inferior portion of the cover defines a gap between the component and the patch,
and wherein preferably, the cover comprises at least one pair of clamping parts arranged at opposing sides of the component, whereby, each of such a pair of clamping parts comprises the inferior portion or a part thereof, which is arranged between the component and the patch.

According to preferred embodiments, the gap is an air gap. According to alternative embodiments the gap may comprise any other suitable medium, such as for example a compressible material configured to provide a cushioning function, such as for example a material comprising silicone rubber.

A further aspect provides a use of a cover and/or a cover assembly as described in the present disclosure for attachment of a component to a patch.

A further aspect provides a use of a patch assembly as described in the present disclosure for measurement of a physiological status of a subject.

### BRIEF DESCRIPTION OF THE FIGURES

The following description of the figures of specific embodiments of the invention are merely exemplary in nature and is not intended to limit the present teachings, their application or uses.

Throughout the drawings, the corresponding reference numerals indicate the following parts and features: patch assembly 1; patch 10; patch layer 11; cover 40; clamping part 41; holding space 42; attachment point 44; proximal portion 45; extension member 46; projection 47; distal projection 471; proximal projection 472; lateral projection 473; aperture 48; membrane 50; component 70; component enclosure 71; membrane component enclosure 75; further component 80; lead 81; clamping force CF; distal direction D; proximal direction P; lateral directions L.
FIG. 1 is a cross-section along line J-J of an embodiment of a patch assembly 1 as shown in the top view of FIG. 2, said patch assembly 1 having a clamping part 41 that exerts a compression force (CF) onto the distal (D) and proximal (P) sides of component 70.
FIG. 2 is a top view of the embodiment of Figure 1.
FIG. 3 is a cross-section of an alternative embodiment of the patch assembly 1 having a clamping part 41 that exerts a compression force (CF) onto all sides of component 70.
FIG. 4 is a cross-section of an alternative embodiment of the patch assembly 1 having a clamping part 41 that exerts a compression force (CF) onto the lateral (L) sides of component 70.
FIG. 5 is a cross-section of an embodiment of the patch assembly 1 having extension members 46.
FIG. 6 is a cross-section of an embodiment of the patch assembly 1 having projections 47.
FIG. 7 is a cross-section of an embodiment of the patch assembly 1 having a distal aperture 48.
FIG. 8 is a cross-section of an embodiment of the patch assembly 1 having a lateral aperture 48.
FIG. 9 is a cross-section of an embodiment of the patch assembly 1 having a plurality of apertures 48.
FIG. 10 is a cross-section of an embodiment of the patch assembly 1 having a membrane 50 disposed in the distal and the proximal portion of the cover 40.
FIG. 11 is a cross-section of an embodiment of the patch assembly 1 having a membrane 50 disposed in the lateral aperture 48 and the proximal portion of the cover 40.
FIG. 12 is a cross-section of an embodiment of the patch assembly 1 having an integrated membrane 50 within cover 40 that fully encloses component 70.
FIG. 13 is a cross-section along line K-K of an embodiment of the patch assembly 1 as shown in the top view of FIG 14, said patch assembly 1 having a further component 80 that is integrated into patch 10.
FIG. 14 is a top view of the embodiment of FIG. 13.
FIG. 15 is a cross-section of an embodiment of the patch assembly 1 having a component enclosure 71.
FIG. 16 is a cross-section of an embodiment of the patch assembly 1 having a membrane component enclosure 75.
FIG. 17 is a cross-section along line L-L of an embodiment of the patch assembly 1 as shown in the top view of FIG. 18, said patch assembly 1 having a further component 80 that is removable from patch 10.
FIG. 18 is a top view of the embodiment of FIG. 17.

### DETAILED DESCRIPTION

The present invention will be described with respect to particular embodiments, but the invention is not limited thereto but only by the claims. Any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" when referring to recited members, elements or method steps also include embodiments which "consist of" said recited members, elements or method steps.

The recitation of numerical ranges by endpoints includes all numbers and fractions subsumed within the respective ranges, as well as the recited endpoints.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

The terms "left," "right," "front," "back," "top," "bottom," "over," "under," "superior," "distal," "inferior," "proximal," "lateral," and the like in the description and in the claims, if any, are used for descriptive purposes and not necessarily for describing permanent relative positions. It is to be understood that the terms so used are interchangeable under appropriate circumstances such that the embodiments described herein are, for example, capable of operation in other orientations than those illustrated or otherwise described herein. "Distal" and "proximal" typically refer to "distal" and "proximal" relative positions with respect to the adhesive patch and/or the skin to which the adhesive patch is applied. "Inferior" and "superior" typically refer respectively to "proximal" and "distal" in that same context.

The term "coupled," as used herein, is defined as directly or indirectly connected in an electrical or nonelectrical (*i*.*e*., physical) manner objects described herein as being "adjacent to" each other may be in physical contact with each other, in close proximity to each other, or in the same general region or area as each other, as appropriate for the context in which the phrase is used.

The term "about" is used to provide flexibility to a numerical range endpoint by providing that a given value may be "a little above" or "a little below" the endpoint. Unless otherwise stated, use of the term "about" in accordance with a specific number or numerical range should also be understood to provide support for such numerical terms or range without the term "about". For example, for the sake of convenience and brevity, when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The term "substantially" refers to the complete or nearly complete extent or degree of an action, characteristic, property, state, structure, item, or result. For example, an object that is "substantially" enclosed would mean that the object is either completely enclosed or nearly completely enclosed. The exact allowable degree of deviation from absolute completeness may in some cases depend on the specific context. However, generally speaking the nearness of completion will be so as to have the same overall result as if absolute and total completion were obtained. The use of "substantially" is equally applicable when used in a negative connotation to refer to the complete or near complete lack of an action, characteristic, property, state, structure, item, or result. For example, a composition that is "substantially free of" particles would either completely lack particles, or so nearly completely lack particles that the effect would be the same as if it completely lacked particles. In other words, a composition that is "substantially free of" an ingredient or element may still actually contain such item as long as there is no measurable effect thereof.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment. Occurrences of the phrase "in one embodiment," or "in one aspect," herein do not necessarily all refer to the same embodiment or aspect. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims and description, any of the claimed or described embodiments can be used in any combination.

In addition, it should be understood that embodiments of the invention may include hardware, software, and electronic components or modules that, for purposes of discussion, may be illustrated and described as if the majority of the components were implemented solely in hardware or software. However, one of ordinary skill in the art, and based on a reading of this detailed description, would recognize that, in at least one embodiment, the electronic based aspects of the invention may be implemented in software (*e*.*g*., instructions stored on non-transitory computer-readable medium) executable by one or more processing units, such as a microprocessor and/or application specific integrated circuits.

Accordingly, the herein described hardware may comprise a processing unit that is configured for executing the herein presented methods as software. Embodiments of the methods may be implemented in code and may be stored on a storage medium having stored thereon instructions which can be used to program a system to perform the instructions. For purposes of the present disclosure, the terms "code" or "program" cover a broad range of components and constructs, including applications, drivers, processes, routines, methods, modules, and subprograms. The terms "code" or "program" may thus be used to refer to any collection of instructions which, when executed by a processing system, performs a desired operation or operations. Additionally, alternative embodiments may include processes that use fewer than all of the disclosed operations, processes that use additional operations, processes that use the same operations in a different sequence, and processes in which the individual operations disclosed herein are combined, subdivided, or otherwise altered. Those skilled in the art can appreciate the numerous modifications and variations thereon.

To provide a solution to the above-described problems in the art, the present disclosure describes a flexible and stretchable cover 40 and/or cover assembly configured for attachment to a patch 10 for attachment to the skin of a subject, for example in medical, lifestyle, wellness, cosmetics, or sports applications, etc. The present cover defines a holding space for one or more components 70, such as for example sensors, processors, data communication modules, batteries, electronic connections, etc. and/or any other suitable electronic components, with the aim of attaching said components to the patch 10. Accordingly, the present disclosure also describes a patch assembly 1 comprising cover 40 and components 70, also referred to as a smart patch.

The mechanical terms "flexible" and "stretchable" as used herein indicate that the referenced component or its material is capable of bending and/or being stretched without breaking when an external force is applied to it and further resuming its former size or shape when said force is released. Nonetheless, the skilled person understands that the flexibility and/or stretchability of any component is defined by the physical properties of its material and structure. Accordingly, to determine whether a given component may be regarded as flexible and/or stretchable within the context of the present disclosure, reference is made to the exemplary embodiments of suitable materials and/or material properties that are discussed further below in the present disclosure. Guided by these exemplary embodiments, the skilled person will be capable of selecting a suitable material and/or structure to produce a component with the desired physical properties, such as a flexible and stretchable cover or any component thereof.

It is clear that in the context of the present disclosure, "flexible and stretchable", when used with reference to the patch 10, refer to material and/or structural properties which lead to a flexibility and/or stretchability that is similar or substantially similar to the flexibility and/or stretchability of the skin to which the patch is configured to be attached. When reference is made to a more "rigid" component 70 in the context of the present disclosure, this refers to a component of which the material properties lead to a flexibility and/or stretchability that is substantially different than that of patch 10 and similarly of the skin to which said patch 10 is configured to be attached to. According to some embodiments, when for example subjected to a similar force, the resulting elongation and/or bending angle of component 70 will be substantially lower than that of the flexible and stretchable patch 10, for example 10% or lower, 5% or lower, or 1% or lower.

Similarly, when "flexible and stretchable" are used in with reference to cover 40, this refers to a flexibility and stretchability caused by suitable material and/or structural properties, which is substantially higher than that of the more "rigid" component 70, and which is similar or at least more aligned with the flexibility and stretchability of the patch 10 and/or skin, than the rigid component. According to some embodiments, when for example subjected to a similar force, the resulting elongation and/or bending angle of the component 70 will be substantially lower than that of the flexible and stretchable cover 40, for example 50% or lower, 25% or lower, or 10% or lower. According to some embodiments, when for example subjected to a similar force, the resulting elongation and/or bending angle of the flexible and stretchable cover 40 will for example be in one or more of the following ranges with respect to that of the patch 10 and/or skin to which the cover is configured to be attached to: 10% up to and including 300%; 30% up to and including 150%; or 50% up to and including 100%.

Component 70 will hereafter be referred to as a single unit, containing all sub-components that enable the intended functioning of said patch assembly 1. Nonetheless, the skilled person may appreciate that component 70 may include various sub-units formed by different components that are optionally in electronic connection with each other. Also, the component may comprise hardware configured for improving the functioning of said electronic components, for example a cooler/heater, humidifier/dehumidifier, etc. For brevity and clarity, similar components are therefore grouped together under one name or descriptor. According to some embodiments the component may comprise and/or consist of an electronic component, a non-electronic component, a reservoir, a dispenser, etc. Such electronic components may for example comprise or consist of one or more electronic elements. Such a non-electric component for example does not comprise any electronic elements. Such a reservoir or dispenser may for example be configured to store and hold a suitable fluid, spray, solid, medicament, cosmetics, etc.

In an embodiment component 70 may comprise an exterior housing that contains and advantageously protects the interior components, which are disposed at least partially within the housing. The housing may be fully rigid but may also be at least partially flexible. Nonetheless, the component housing will typically be more rigid, less flexible than cover 40 in order to adequately protect component 70.

Patches 10 for attachment to skin of a subject are known in the art. For example, a patch 10 may be self-adhesive, *e*.*g*., disposed with a skin-adhesive layer on the proximal, *i.e.,* skin facing side, or may require the provision of an adhesive on the subject skin. Advantageously, patch 10 consist of a compliant material configured to conform to a shape of the skin and/or changes to said skin shape during movements of the subject. Further advantageous embodiments of a patch 10 in relation to the present cover 40 will be clarified throughout the present disclosure.

The subject may be a mammal more specifically a human, such as a healthy human or a patient suffering from an abnormal health condition. The cover 40 and/or patch assembly 1 may be assigned to a single subject, for example in a single-use and/or partially disposable embodiment, but if so desired, can be repurposed for use by another subject or group of subjects, for example in a partially or fully reusable embodiment as will become clear from the description below.

An initial overview of various components of the cover is provided below and specific embodiments are then described in further detail. This initial overview is intended to aid readers in understanding the technological concepts more quickly but is not intended to identify key or essential features thereof, nor is it intended to limit the scope of the present subject-matter.

In addition, one of ordinary skill in the art understands, and based on a reading of this detailed description, would recognize that the various aspects can be combined unless otherwise stated. As such, any specific embodiment of a specific aspect may be understood to constitute a specific embodiment of another aspect without the explicit discussion thereof. For example, an embodiment for the configuration of the cover also forms an embodiment for the manufacture of the cover according to said configuration, an embodiment for the use of said cover according to said configuration, and so on.

Cover 40 consists of a flexible and stretchable material that forms a barrier configured to at least partially enclose component 70. The interior of the cover defines a holding space for holding the component. For this purpose, cover 40 may comprise a lateral portion configured to at least partially cover a lateral side of component 70, a distal or superior portion configured to at least partially cover a distal or superior side of component 70, and/or a proximal or an inferior portion 45 configured to at least partially cover a proximal or an inferior side of component 70.

In an embodiment cover 40 may comprise a lateral portion configured to completely cover a lateral side of component 70, a distal portion configured to completely cover a distal side of component 70, and/or a proximal portion 45 configured to completely cover a proximal side of component 70. It is understood that a complete cover of a side of component 70 also includes an almost complete cover notwithstanding the provision of apertures or pores allowing the passage of gas/fluids.

The flexible and stretchable material of cover 40 may act as a flexible stop member that provides resistance against displacement of component 70 in at least one direction; component 70 may still move, but the movement requires more force against the resistance defined by the material properties of cover 40. Accordingly, the provision of a more flexible and stretchable material may allow for greater displacement compared to a less flexible and stretchable material. Nevertheless, as is clear from the present disclosure it is an object of cover 40 to prevent a removal of component 70 from the patch and hence a limit on the maximal displacement is implied.

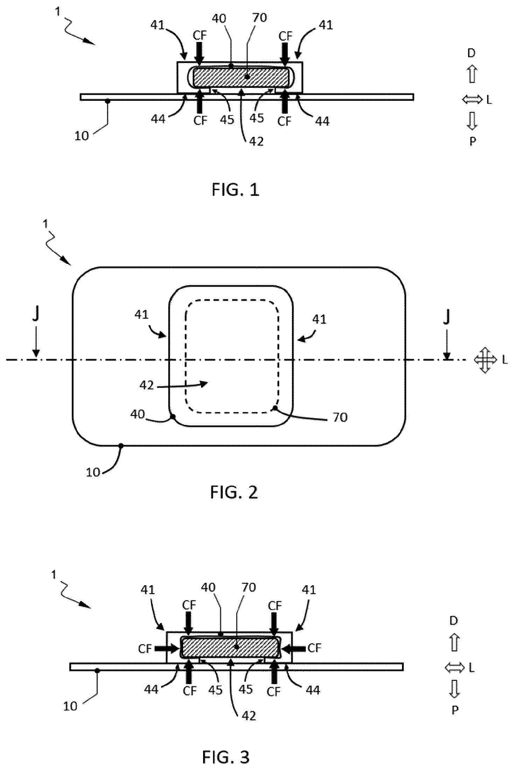

**Figure 1** shows an embodiment of cover 40 comprising a flexible and stretchable clamping part 41 configured to clamp component 70 when mounted inside cover 40 in such a way that the clamping part 41 is in contact with component 70 and applies a compression force CF to the component attaching component 70 to the inside of cover 40. Accordingly, **Figure 1** also shows a cover assembly comprising cover 40 and component 70 wherein said component 70 is mounted inside the cover 40 by means of the at least one flexible and stretchable clamping part 41.

Component 70 may be mounted into cover 40 prior to being clamped by the clamping part 41 for easier mounting/dismounting of component 70 from cover 40. In such an embodiment cover 40 may be provided with a clamping mechanism to initiate the compression force exerted by clamping part 41 onto component 70. Accordingly, clamping part 41 may be configured to exert a compression force onto the component, thereby attaching component 70 to the inside of cover 40.

Compression force or compressive force as used herein refers to a physical force pressing inward on an object, such as the component 70, causing it to become attached. The compression force may limit movement of said component 70 in at least one direction, preferably a distal (D) or superior, and/or a proximal (P) or inferior direction, yet possibly still allowing a sliding movement of the component in another, preferably lateral (L) direction with the cover material acting as a flexible stop member.

As will become clear from embodiments described further on, attachment of component 70 may be temporary or permanent depending on an embodiment of clamping part 41. For example, component 70 may need to be displaced *e*.*g*., twisted, turned, bended, etc., into a specific position within the holding space 42 for the clamping part 41 to clamp said component 70. Alternatively, in an embodiment component 70 may be automatically clamped by clamping part 41 during insertion or mounting resulting in easier and faster attachment thereof. Also, there can be different results depending on the direction or position on the object that the compressive force is applied to.

In an embodiment clamping part 41 may be configured such that, when subjected to changes resulting from flexing and/or stretching of cover 40, the material of clamping part 41 in contact with the component 70 is slidable with respect to component 70, *i*.*e*., component 70 can slidably move within the holding space 42 of cover 40. Preferably the surface of clamping part 41 has a coefficient of friction suitable for allowing a sliding motion of component 70 when a force is applied onto the cover 40. It is appreciated that the suitable friction coefficient is dependent on the contacting surface materials of clamping part 41 and component 70. The skilled person can select a suitable material guided by the exemplary embodiments discussed further below.

In another embodiment the clamping part 41 may be configured such that, when subjected to changes resulting from flexing and/or stretching of the cover 40, the material of the clamping part 41 in contact with the component 70 is adherent with respect to component 70; *i*.*e*., component 70 is fixed within the holding space 42 of cover 40. Preferably the material of clamping part 41 has a coefficient of friction that is suitable for preventing most if not all motion of component 70. The skilled person can select a suitable material.

In another embodiment the clamping part 41 may be configured such that, when subjected to changes resulting from flexing and/or stretching of the cover 40, the material of the clamping part 41 in contact with the component 70 is slidable with respect to component 70 in a direction but adherent in another direction. For example, the component 70 can slidably move in a lateral direction but is fixed in a distal/proximal direction, or vice versa.

The above-described embodiments notwithstanding, in practice the clamping part 41 materials may have a slidability/adherence that is dependent on the force exerted onto component 70. In other words, the clamping part 41 may provide for an embodiment that is adherent when a relatively minor force is applied, such that component 70 remains fixed in place, but is slidable to a degree when a relatively major force is applied, such that component 70 can move. This way the position of component 70 within the holding space 42 can be adjusted when desired, for example by being grabbed or pushed, but does not move during normal movement of the subject. It is therefore understood that in a preferred embodiment no other means for attaching component 70 to cover 40 are provided, such as an adhesive substance applied between component 70 and cover 40, or a fastener disposed through or around cover 40 to fasten the component 70 within. Accordingly, cover 40 may be considered as the primary means for attaching component 70 to patch 10 within the holding space of cover 40, *i*.*e*., without the provision of other means *e*.*g*., adhesives, fasteners, or the like. It is understood that the means for attaching excludes any components that are not intended to attach component 70. For example, component 70 may include various electrical components such as leads or cables that connect it to another component 80 in the assembly 1, such as one or more sensors. Nonetheless, such electrical components are typically not configured to fasten said component 70 and may therefore be damaged or broken when component 70 is moved, or alternatively, may be configured to automatically disconnect to prevent damage. The provision of a "clamping only" attachment can reduce the complexity of the design, and advantageously enable displacement of component 70 in accordance with an embodiment of cover 40 and/or promote easier passage of gas/fluids from the subject's skin through cover 40.

In an embodiment clamping part 41 may be configured to apply a compression force to component 70 in accordance with at least one of the following options:
- along at least one direction, *e*.*g*., distal or proximal direction;
- along a plurality of directions, *e*.*g*., distal and proximal directions, lateral directions;
- inwards along at least a part of an outer surface of component 70.

With reference to **Figure 1****,** an embodiment of cover 40 is shown wherein the clamping part 41 exerts a compression force (illustrated by the four CF arrows) onto the distal D and proximal P sides of the component, thereby limiting movement of said component 70 in the distal/proximal directions. The illustrated compression force can be realised by simultaneously compressing a distal side of component 70 from a distal portion of cover 40 and a proximal side of component 70 with a proximal portion 45 of cover 40. In the present embodiment no compression force is exerted onto the lateral L sides of component 70, such that it can potentially move in the lateral direction by sliding within the cover's holding space 42 up to the lateral portion of cover 40 that forms a flexible stop member.

With reference to **Figure 3****,** an alternative embodiment of cover 40 is shown wherein the clamping part 41 exerts a compression force (illustrated by the six CF arrows) onto the distal D and proximal P sides and the lateral L sides of the component, thereby limiting movement of said component 70 in the distal, proximal and lateral directions. The illustrated compression force is realised by simultaneously compressing a distal side of component 70 with a distal portion of cover 40, a proximal side of component 70 with a proximal portion 45 of cover 40, and the lateral sides of component 70 with the lateral portions of cover 40. In the present embodiment the clamping part 41 adheres to component 70 such that it can prevent movement of component 70 within the cover's holding space 42.

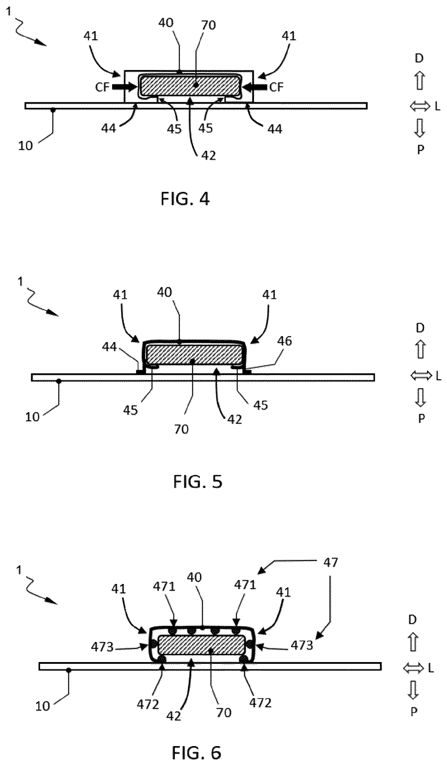

With reference to **Figure 4****,** another alternative embodiment of cover 40 is shown wherein the clamping part 41 exerts a compression force (illustrated by the two CF arrows) onto the lateral L sides of component 70, thereby limiting movement of said component 70 in the lateral directions, and advantageously also the distal and/or proximal directions. The illustrated compression force is realised by simultaneously compressing the lateral sides of component 70 with the lateral portions of cover 40. In an embodiment at least two opposite lateral portions of cover 40, *i*.*e*., on opposite sides of the cover 40, may apply a compression force. In a further embodiment at least four lateral portions of cover 40, *i*.*e*., two by two opposite sides of the cover 40 may apply a compression force. The skilled person understands that for embodiments wherein the cover has a divergent shape, for example hexagonal or octagonal, multiple lateral portions may apply a compressive force onto component 70. Also, in the present embodiment no compression force is exerted onto the proximal and the distal sides of component 70, which can therefore move in the proximal and/or distal direction by sliding within the holding space up to the distal portion of cover 40 that forms a flexible stop member.

Preferably the exerted compression force may be applied at different positions along at least one side of component 70 to improve the clamping reliability. For example, with reference to **Figure 1** an embodiment is illustrated wherein the compression force is exerted at opposite edges of component 70, specifically at a proximal edge and a distal edge of cover 40. Advantageously, the compression force may be exerted along at least a part of, preferably an entire side, of component 70. For example, with reference to **Figure** 3 an embodiment is illustrated wherein the compression force is continuously exerted along the lateral sides of component 70, specifically along two opposing lateral side of component 70. The skilled person understands that the embodiment of **Figure** 3 is a cross-section (for example along the length of the assembly) and the non-illustrated lateral sides of cover 40 may similarly exert a compression force onto component 70 such that the compression force is exerted along the entire lateral side of component 70.

In an embodiment the cover 40, specifically the clamping part 41, may be configured to grip at least part of component 70. Advantageously at least one the clamping part 41 may be provided with a rigid member extending from cover 40 that can grip component 70 when it is inserted or mounted inside cover 40. Gripping of component 70 may be particularly advantageous for embodiments wherein cover 40 has a more limited contact surface with component 70, such as embodiments comprising a relatively large and/or multiple apertures, to prevent release of said component 70.

Referring back to **Figure 1****,** an embodiment of cover 40 is shown comprising an attachment point 44 configured for attachment to a patch 10, *i*.*e*., a patch attachment point 44. In the present embodiment cover 40 is shown having two attachment points 44 arranged on opposite sides of component 70. However, additional attachment points 44 may be provided at fixed positions along an attachment surface of patch 10. In an embodiment a continuous attachment may run continuously along the edges of cover 40 to provide a continuous attachment surface to patch 10. The skilled person may therefore appreciate that the provision of more attachment points may strengthen the attachment of cover 40 to patch 10.

In an embodiment attachment point 44 may be non-removably attached to patch 10 to provide secure attachment thereto. For example, attachment point 44 may be attached to patch 10 by means of an adhesive or other suitable fixating methods, such as welding, overmoulding, etc.

In an embodiment attachment point 44 may be removably attached to patch 10 for easier mounting/dismount of component 70 to patch 10. For example, attachment point 44 may be provided with a reusable mechanism such as a hook and loops. The removable attachment may provide for a partially reusable embodiment of the patch assembly 1, for example wherein patch 10 may be disposable and cover 40 reusable, or vice versa. Advantageously, patch 10 may be provided with an attachment means configured for mutual attachment to one or more corresponding attachment points 44 of cover 40.

Referring back to **Figure 1** an embodiment of cover 40 is shown wherein the proximal portion 45 is disposed adjacent to component 70 optionally spaced apart from patch 10 so as to define a gap between said component 70 and said patch 10. The gap may provide space between component 70 and patch 10 preventing the subject's skin from pushing against component 70 which might cause patch 10 to detach from the skin. Moreover, the gap may allow easier passage of gas/fluids necessary for breathing of skin, *e*.*g*., "breathable" patch. As such, wearing comfort for the subject can be improved which is particularly beneficial for applications requiring prolonged wearing of the patch 10. In some preferred embodiments the gap has a depth, defined as the distance from the skin facing proximal side of component 70 and the component facing distal side of patch 10, ranging from at least 0.1 mm to at most 20.0 mm; preferably 0.5 mm to 15.0 mm; more preferably 0.5 mm to 10.0 mm; even more preferably 1.0 mm to 5.0 mm; even more preferably 1.5 mm to 2.5 mm; even more preferably 1.5 mm to 2.5 mm; even more preferably about 2.0 mm. It may be appreciated that the optimal depth of the gap is dependent on the size of component 70, in particular its length along the patch 10, and the location of the patch 10 on the subject's skin. Nevertheless, the listed ranges were identified as suitable for a general-purpose patch assembly 1.

In an embodiment cover 40 may comprise a breathing aperture configured for allowing the passage of gas and optionally fluid therethrough, *i*.*e*., "breathable" cover. In a preferred embodiment the breathing aperture may be pore or (micro)perforation provided in the surface of cover 40. Typically, a plurality of such breathing apertures preferably pores will be provided, of same or different sizes, which may be arranged in a specific shape or form to promote efficient passage of gas and/or fluid through specific regions or parts of the patch assembly 1. Advantageously, the aperture preferably pore may be in fluid connection to the above-described gap such that gas and optionally fluid can flow through cover 40. In an example this may enable gas exchange between the subject's skin and patch exterior so that humidity or condensation on component 70 may be prevented. In another example this may allow the supplying of gas such as air necessary for a battery contained within the patch assembly for example on component 70. Advantageously the aperture preferably pore has a relatively small diameter to substantially not affect the material properties of cover 40 or form structural weak spot that could cause said cover 40 to tear or wear more easily.

In a particular embodiment the "breathable" cover may be formed through a combination of one or more apertures which are covered with or contain a membrane 50 disposed within, which membrane 50 is selectively permeable to gas and/or impermeable to fluid. Optionally such a membrane 50 may consist of a material containing one or more pores. Embodiment of a suitable membrane 50 are discussed further in the present disclosure.

**Figure 5** shows an embodiment of cover 40 comprising one or more extension members 46 configured to determine the position of the holding space 42 in a distal direction from the one or more points of attachment 44. The extension members can additionally ensure that component 70 is kept at a minimal distance from the subject's skin during movement of the subject. Advantageously the extension members 46 are rigid enough to resist the weight of component 70 without completely deforming.

In a particular embodiment the extension members 46 further determine the depth of the gap between component 70 and patch 10. The provision of extension members 46 thereby provides a simple solution to ensure a minimal gap depth according to an embodiment as discussed in the present disclosure. As such, the length of extension member 46 can determine the size of the gap in 3 dimensions, *i*.*e*., the gap depth, width and length.

In some embodiments the extension member 46 has a length, defined as the distance from the attachment point 44 and the clamping part 41 or a proximal portion 45 thereof, ranging from at least 0.1 mm to at most 20.0 mm; preferably 0.5 mm to 15.0 mm; more preferably 0.5 mm to 10.0 mm; even more preferably 1.0 mm to 5.0 mm; even more preferably 1.5 mm to 2.5 mm; even more preferably 1.5 mm to 2.5 mm; even more preferably about 2.0 mm. It may be appreciated that the optimal length of extension member 46 is dependent on the size of component 70, in particular its length along the patch 10, and the location of the patch 10 on the subject's skin. Nevertheless, the listed ranges were identified as suitable for a general-purpose patch assembly 1.

**Figure 6** shows an embodiment of cover 40, having an interior surface facing component 70 and an opposing exterior surface, wherein the interior surface is disposed with a plurality of projections 47 extending from the interior surface that are preferably in slidable contact with component 70.

The projections 47 provide a spacing between the inner surface of cover 40 and component 70. In a generic embodiment, projections 47 may be configured to further limit/enable slidable movement of component 70 within cover 40. The projections 47 may also reduce the size of the contact area between the adjacent surfaces of cover 40 and component 70, thereby reducing friction on the component surface when sliding in a direction within the holding space. Accordingly, the projections 47 may be configured to provide a flexibility and stretchability that is different from the cover 40 interior material. Also, the projections 47 may act as a "bumper" by absorbing shock or preventing impact damage from the exterior.

The projections 47 may have any geometrical shape. The projections 47 shown in **Figure 6** have a spherical shape and it is understood that this forms a preferred embodiment of the projections. Nevertheless, other geometrical shapes may be equally viable, such as cylinders, pyramids, screws, and so on.

With further reference to **Figure 6****,** it is shown that there may be provided projections on different portions of cover 40; namely, a projection 47 on the interior surface of the distal portion of cover 40, referred to as distal projection 471; a projection 47 on the interior surface of the proximal portion of cover 40, referred to as proximal projection 472; and a projection 47 on the interior surface of the lateral portion of cover 40, referred to as lateral projection 473. Each one of these projections 47 has a particular advantage, in addition to the above-discussed advantages, which will be discussed below. The present embodiment notwithstanding, it is understood that each discussed projection 47 may be provided without or in combination with the other discussed projections 47. For example, only proximal projections 472, only distal 471 and proximal projections 472, and so on.

The distal projection 471 may further maintain a gap between the interior surface of cover 40 and component 70. This gap may ensure that oxygen flow to the batteries (*e*.*g*. zinc-air batteries), which could otherwise be sealed off by said cover 40.

The proximal projection 472 may further determine the depth of the gap between component 70 and patch 10. The provision of extension members 46 thereby provides a simple solution to ensure a minimal gap depth according to an embodiment as discussed in the present disclosure. As such, the diameter of proximal projection 472 can determine the size of the gap in 3 dimensions, i.e., the gap depth, width and length. However, contrary to the above discussed extension member 46, the proximal projection 472 may be provide higher flexibility and stretchability and as such increase the depth of the gap with lower impact on the flex and stretch of cover 40.

The lateral projection 473 may further protect component 70 against sudden impacts during use of patch assembly 1 and/or allow easier handling of said patch, for example when inserting component 70 or adjusting cover 40.

As will be further discussed in the present disclosure, the component 70 may be provided with a component enclosure 71 that in an embodiment may be provided with at least one or more projections 47 according to the above discussed embodiments thereof. The skilled person may therefore appreciate that similar advantages may be achieved depending on the parameters and location of the provided projections 47. In a further embodiment a plurality of projections 47 may be provided on the cover 40 and component 70, which are configured to achieve a combined effect with regard to further limiting/enabling slidable movement of component 70 within cover 40.

In some embodiments the projection 47 has a height, or diameter in case of a spherical embodiment, ranging from at least 0.1 mm to at most 2.0 mm; preferably 0.2 mm to 1.9 mm; more preferably 0.3 mm to 1.8 mm; even more preferably 0.4 mm to 1.7 mm; even more preferably 0.4 mm to 1.6 mm; even more preferably 0.5 mm to 1.5 mm; even more preferably 0.6 mm to 1.4 mm; even more preferably 0.7 mm to 1.3 mm; even more preferably 0.8 mm to 1.2 mm; even more preferably 0.9 mm to 1.1 mm; even more preferably about 1.0 mm. It may be appreciated that the optimal height/diameter of projection 47 is dependent on the dimensions of component 70, in particular its height, length and width, and the location of the patch 10 on the subject's skin. Nevertheless, the listed ranges were identified as suitable for a general-purpose patch assembly 1.

In a particular embodiment the different projection 47 may have different material properties depending on the position in cover 40. In an exemplary embodiment the proximal projection 472 can be made more rigid to provide an improved resistance to pressure and better maintain the gap size, whereas the distal 471 or lateral projections 473 can be made more flexible to provide an improved shock resistance.

In an embodiment cover 40 may comprise an aperture 48 configured for accessing and/or inserting the component 70 or a part thereof. The diameter of said aperture 48 may be adjustable, preferably stretchable, to allow easier access or insertion. Advantageously, the aperture may be disposed on the distal and/or proximal portion 45 of cover 40 to avoid having a structural weak spot in the lateral portion of cover 40. Nonetheless, an embodiment having an aperture disposed on a lateral portion of cover 40 may be contemplated provided that the compressive force exerted by clamping part 41 is not compromised that could result in an accidental release of component 70.

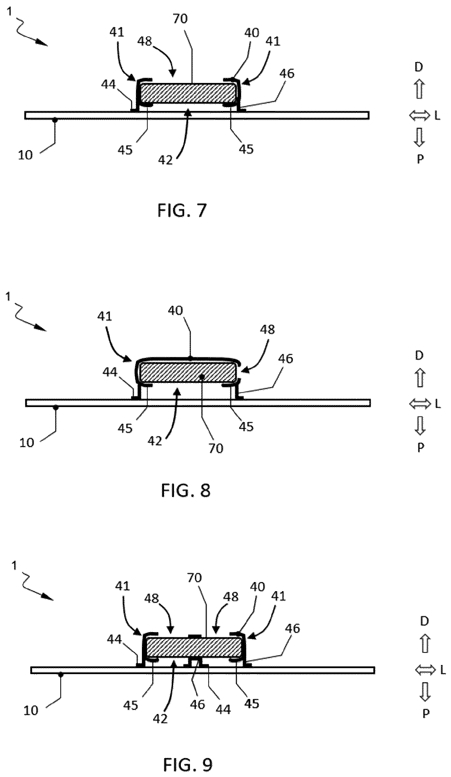

With reference to **Figure 7****,** an embodiment of cover 40 is shown wherein an aperture 48 is provided on the distal portion of cover 40. The large diameter of aperture 48 may allow easier access to component 70, for example for insertion or access of a part thereof, such as a screen or other user input means. The clamping part 40 may comprise an extension member that holds a part of the distal portion of component 70 such that it can be gripped within cover 40 to prevent accidental release. Advantageously clamping part 40 exerts a proximal compression force onto component 70.

With reference to **Figure 8****,** an embodiment of cover 40 is shown wherein an aperture 48 is provided on the lateral portion of cover 40. The side position of aperture 48 may allow easy sideways insertion of component 70 without compromising on the integrity of cover 40. Advantageously the attachment point 44 and optional extension member 46 adjacent to aperture 48 may be reinforced to ensure that cover 40 does not deform during inserting of component 70. For example, the cover material can be made more rigid around aperture 48.

With reference to **Figure 9****,** an embodiment of cover 40 is shown wherein a plurality of apertures 48 is provided on the distal portion of cover 40. In the shown embodiment two apertures 48 are provided on said distal portion, but it is understood that the cover 40 can be easily adapted to have more than two apertures 48, such as three, four or more apertures 48. Advantageously the plurality of apertures may be arranged in a specific shape or form, for example to allow easier access to specific portions of component 70.

It is further shown in Figure 9 that the proximal portion of cover 40 may be divided by an additional support member comprising an attachment point 44 and/or extension member 46. This allows for dividing the proximal portion of cover 40 into two or more parts such that the stability and/or rigidity of cover 40 may be further improved. In the shown embodiment one such support member is provided centrally below component 70, but it is understood that the cover 40 can be easily adapted to have more than one support member, such as three, four or more, which may be arranged at different positions on the proximal portion of cover 40.

In an embodiment patch assembly 1 may comprise a membrane 50 which is at least partially permeable to gas. The membrane 50 may be selectively permeable to gas, such as only allowing air or specifically oxygen/carbon dioxide to pass, or it may be generally permeable to allow free passage of air. Such a membrane may allow thereby passage of gas necessary for breathing of skin, *e*.*g*., "breathable" patch, to prevent condensation on the patch 10 and/or component 70.

Typically, a membrane 50 may comprise a pore or (micro)perforation configured for to allow the passage of gas and optionally fluid therethrough, *i*.*e*., "breathable" membrane. Typically, a plurality of pores will be provided, which may be arranged in a specific group or shape to promote efficient passage of gas and/or fluid through the patch assembly 1. In a preferred embodiment the pores will be configured to allow free or selective passage of air but block passage of fluid.

In an embodiment membrane 50 may have one or more pores with a pore size in the range of at least 0.1 µm to at most 10.0 µm; preferably 0.1 µm to 9.0 µm; more preferably 0.1 µm to 8.0 µm; even more preferably 0.1 µm to 7.0 µm; even more preferably 0.1 µm to 6.0 µm; even more preferably 0.1 µm to 5.0 µm; even more preferably 0.1 µm to 4.0 µm; even more preferably 0.1 µm to 3.0 µm; even more preferably 0.2 µm to 3.0 µm; even more preferably 0.3 µm to 3.0 µm; for example, 0.5 µm, 1.0 µm, 1.5 µm, 2.0 µm, or 2.5 µm.

In an embodiment membrane 50 may have a pore density of at least 10⁵ to at most 10⁹ pores/cm²; preferably 10⁶ to at most 10⁸ pores/cm². It may be appreciated that the optimal pore size and pore density are dependent on the required air flow rate, which in turn depends on the application and location of the patch 10 on the subject's skin. Nevertheless, the listed ranges were identified as suitable for a general-purpose patch assembly 1.

In an embodiment membrane 50 may have a thickness of at least 1 µm to at most 500 µm; preferably 2 µm to 400 µm; more preferably 5 µm to 300 µm; even more preferably 10 µm to 200 µm.

Membrane 50 will typically be disposed within the patch assembly 1 at a location where passage of gas is desired. Accordingly, it may be disposed between patch 1 and component 70 and partially cover component 70. For example, membrane 50 may be disposed within the gap as described above, or membrane 50 may be arranged around component 70 and at least partially enclosed said component 70. Below exemplary embodiments will be discussed with membrane 50. It is understood that these embodiments can be easily expanded or combined.

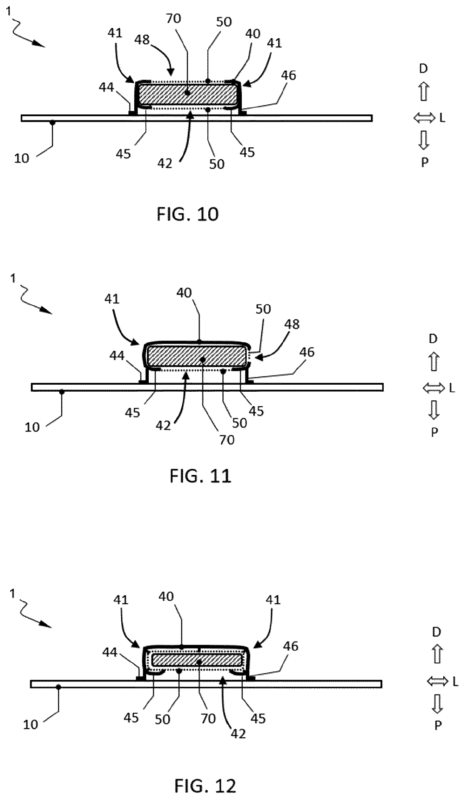

With reference to **Figure 10****,** an embodiment of cover 40 is shown wherein a membrane 50 is disposed in the space between patch 10 and component 70 on the proximal portion of cover 40. This proximal membrane 50 may be connected to the edges of cover 40, preferably on the proximal portion 45 of cover 40. Alternatively, proximal membrane 50 may be connected to other portions of cover 40, such as attachment point 44 and/or extension member 46. Nonetheless, providing some space between patch 10 and membrane 50 and/or component 70 and membrane 50 may allow for easier passage of gas and/or fluid to achieve sufficient skin ventilation underneath component 70.

It is further shown in **Figure 10** that another membrane 50 is disposed in an aperture 48 provided on the distal portion of cover 40. This distal membrane 50 may be connected to the edges of cover 40, preferably on the distal portion of cover 40. The distal membrane 50 may enable gas exchange between holding space 42 and the exterior air while protecting component 70 for example from external fluids that might leak into holding space 42. In a preferred embodiment distal membrane 50 may be provided with breathable pores as discussed previously in the present disclosure. It is understood that the distal and proximal membranes 50 are shown in a single preferred embodiment but the cover may be easily adjusted to contain a membrane at one side only, such as only a distal membrane 50 or only a proximal membrane 50. The shown embodiment having both distal and proximal membranes 50, however, has the added benefit of allowing gas, for example air, to pass through the membrane 50 from and/or to a plurality of sides of cover 40, while protecting the component 70 from water or other fluids. This will for example a better flow of gas through the cover, which as for example mentioned above provides for an improved breathability with respect to the skin and/or is provides for the oxygen to allow for the operation of suitable batteries.

With reference to **Figure 11****,** an embodiment of cover 40 is shown wherein a membrane 50 is provided on the lateral portion of cover 40. This lateral membrane 50 may be connected to the edges of cover 40, preferably on the lateral portion of cover 40. Similar to the above-discussed membranes, the lateral membrane 50 may enable gas exchange between holding space 42 and the exterior air. The lateral membrane 50, however, has the added benefit that it is not cut off from air when the subject or another object covers the distal portion of cover 40, for example when lying down.

With reference to **Figure 12****,** an embodiment of cover 40 is shown wherein a membrane 50 is disposed around component 70 to enclose it. As illustrated, the membrane 50 may be connected to cover 40 through connective members extending from said cover 40 towards component 70. Alternatively, membrane 50 may be directly connected to cover 40, such that it becomes fully integrated within said cover 40. Such an embodiment is advantageous to completely protect component 70 from *e.g.,* fluids or condensation. It is understood that embodiments wherein membrane 50 only partially encloses component 70 are also anticipated. For example, in an embodiment as shown in **Figure 7** wherein cover 40 is provided with a distal aperture 48, the membrane 50 may be disposed at a distal portion thereof.

In a further embodiment the patch assembly 1 may comprise a plurality of membranes 50. For example, a first membrane provided between patch 10 and component 70, which is partially permeable for fluids, and a second membrane provided between cover 40 and component 70, which is impermeable for fluids. The stacking of membranes may allow for a selective passage of gas and/or fluids from the skin to the exterior, such as sweat.

In a particular embodiment the membrane 50 may consist of or be replaced by a modified segment of cover 40, which is preferably thinner than the average cover wall thickness and is provided with a plurality of pores or micro(perforations) that are limited to the said modified segment. The advantage of such an embodiment is that a continuous cover 40 can be produced to prevent the presence of structural weak spots that might occur at the transition of cover 40 into membrane 50. For example, in an embodiment the cover may consist of silicone rubber that has a thinner segment of slitted silicone rubber that effectively forms a membrane on said cover 40. It is understood that other cover materials are also regarded as suitable for such an embodiment.

In an embodiment the cover 40 material has a hardness, defined by a Shore A hardness value as measured in accordance with ISO 7619-1, in the range of at least 10 shore A to at most 90 shore A; preferably 20 shore A to 80 shore A; more preferably 30 shore A to 70 shore A; even more preferably 30 shore A to 60 shore A; for example, 40 shore A or 50 shore A. It may be appreciated that the optimal hardness is dependent on the adherence or slidability of the desired embodiment. Typically, the lower the hardness value, the higher the adherence of the material and the lower its slidability. The skilled person can select a suitable material for its intended purpose.

In an embodiment the cover 40 material has a tensile strength, as measured in accordance with ISO 37 type 1, in the range of at least 5.0 N/mm² to at most 11.0 N/mm²; preferably 5.5 N/mm² to at most 10.5 N/mm²; more preferably 6.0 N/mm² to at most 10.0 N/mm²; even more preferably 6.5 N/mm² to at most 9.5 N/mm²; even more preferably 7.0 N/mm² to at most 9.0 N/mm²; even more preferably 7.5 N/mm² to at most 8.5 N/mm²; even more preferably about 8.0 N/mm², for example 8.2 N/mm² or 8.5 N/mm². It may be appreciated that the optimal tensile strength is dependent on the rigidity or stretchability of the desired embodiment. The skilled person can select a suitable material for its intended purpose.

In an embodiment the cover 40 material has an elongation, defined by the maximal elongation at break as measured in accordance with ISO 37 type 1, in the range of at least 100% to at most 800%; preferably 200% to 800%; more preferably 300% to 750%; even more preferably 350% to 750%; even more preferably 400% to 700%; even more preferably 450% to 650%; even more preferably 500% to 600%; for example, 550% or 590%. It may be appreciated that the optimal elongation is dependent on the rigidity or stretchability of the desired embodiment. The skilled person can select a suitable material for its intended purpose.

In an embodiment the cover 40 material has a tear strength, as measured in accordance with ASTM D 624 BN, in the range of at least 20 to at most 30 N/mm; preferably 21 to 29 N/mm; more preferably 21 to 28 N/mm; even more preferably 22 to 27 N/mm; even more preferably 23 to 26 N/mm; for example, 24 N/mm or 25 N/mm. It may be appreciated that the optimal tear strength is dependent on the rigidity or stretchability of the desired embodiment. The skilled person can select a suitable material for its intended purpose.

In an embodiment cover 40 material comprises, preferably consists of, silicone rubber(SR), more preferably liquid silicone rubber (LSR); a thermoplastic elastomer (TPE), more preferably thermoplastic polyurethane (TPU), thermoplastic olefin (TPO), thermoplastic polyamide (TPA); a thermoplastic vulcanizate (TPV), a cross-linkable elastomer, more preferably isoprene rubber (IR), nitrile rubber (NBR), styrene-butadiene rubber (SBR), Ethlylene Propylene Diene Monomer (EPDM), fluorocarbon-based fluoroelastomer materials (FKM), polybutadiene rubber (BR); and/or combinations thereof.

In an embodiment cover 40 may be transparent to allow easier visual tracking of the position of component 70. Nonetheless, the provision of pigments to add colour to the cover 40 may also be considered depending on the intended application.

For convenience and clarity, cover 40 was discussed in the present disclosure as a monolayer consisting of a single material. Such simple embodiments notwithstanding, it may be appreciated that cover 40 may easily be multi-layered and/or consist of a combination of materials dependent on the rigidity or stretchability of the desired embodiment. For example, a multi-layered cover 40 may have an exterior layer, facing the exterior space, which is more rigid to better resist external force, and an interior layer, facing component 70, which is more flexible or stretchable for the previously discussed advantages, or vice versa. In another example, a multi-layered cover 40 may be composed of different materials at specific parts of cover 40; for example, the corners may be made more rigid and the side walls in contact with component 70 may be more flexible or stretchable, or vice versa; for example, the lateral portions of cover 40 may be made more rigid and the lateral portions the distal and proximal portions may be more flexible or stretchable, or vice versa; for example, the clamping part 41, attachment point 44, extension member and/or projection 47 can be made of same or different materials depending on their desired properties. The skilled person can select a suitable combination of materials and/or layers for its intended purpose.

In an embodiment patch assembly 1 may comprise a component enclosure 71 at least partially enclosing component 70. The component enclosure 71 may provide a protective barrier to protect and/or secure the component 70 or parts thereof within cover 40. Additionally, the component enclosure 71 may consist of a material that provides a coefficient of friction that is suitable for achieving a selected embodiment of the patch assembly 1. For example, the component enclosure 71 may have a coefficient of friction that enable a sliding motion of component 70, which would otherwise be difficult or impossible with the exterior material of said component 70. Alternatively, the protective enclosure 71 may have a coefficient of friction to adhere to cover 40 in another embodiment thereof. As such, the skilled person understands that the component enclosure 71 may be rigid, flexible, or somewhere in-between depending on the embodiment. Also, the component enclosure 71 may be composed of parts that are rigid, for example the corners, and parts that are flexible, for example along the side walls. It is understood that these embodiments can be easily expanded or combined.

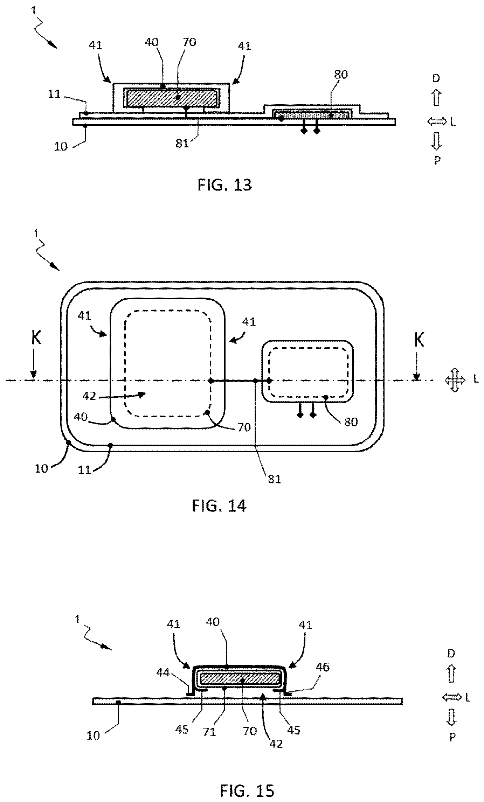

With reference to **Figure 15****,** an embodiment is shown wherein a component enclosure 71 is disposed in in the holding space 42 between cover 40 and component 70 to fully enclose said component 70. It is understood that embodiments wherein membrane 50 only partially encloses component 70 are also anticipated, for example if clamping part 41 applies a compression force in the lateral directions only, a component enclosure 71 enclosing the lateral portions of said component 70 may be sufficient. Likewise for a distal/proximal compression force.

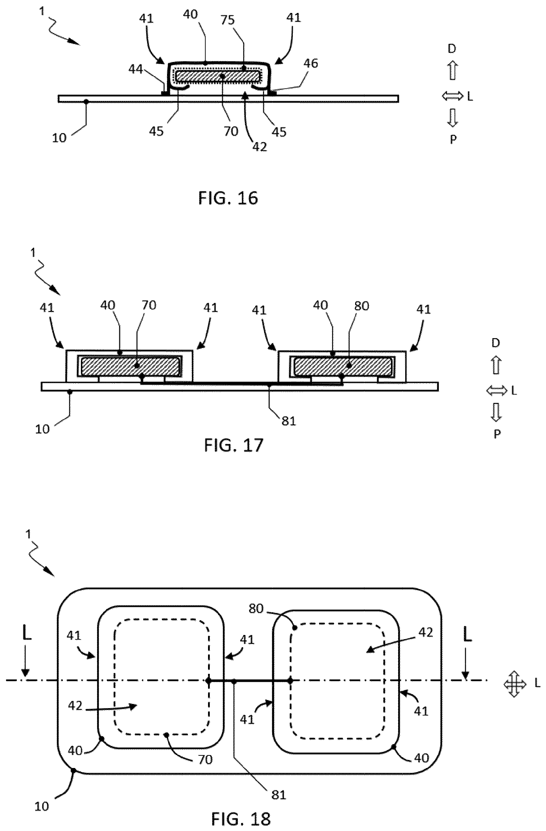

With reference to **Figure 16****,** an embodiment is shown wherein a membrane is integrated into the component enclosure thereby forming a membrane component enclosure 75. It is understood that the membrane component enclosure 75 may be provided in the same embodiments as described above for the component enclosure 75 with the addition of a membrane. For example, the membrane component enclosure 75 may only partially enclose component 70 with a membrane, for example only a distal or proximal portion of component 70, or fully enclose component 70 with a membrane.

In an embodiment the component enclosure 71 material has a hardness, defined by a Shore A hardness value as measured in accordance with ISO 7619-1, in the range of at least 10 shore A to at most 90 shore A; preferably 20 shore A to 80 shore A; more preferably 30 shore A to 70 shore A; even more preferably 30 shore A to 60 shore A; for example, 40 shore A or 50 shore A.

In an embodiment the component enclosure 71 material has a tensile strength, as measured in accordance with ISO 37 type 1, in the range of at least 5.0 N/mm² to at most 11.0 N/mm²; preferably 5.5 N/mm² to at most 10.5 N/mm²; more preferably 6.0 N/mm² to at most 10.0 N/mm²; even more preferably 6.5 N/mm² to at most 9.5 N/mm²; even more preferably 7.0 N/mm² to at most 9.0 N/mm²; even more preferably 7.5 N/mm² to at most 8.5 N/mm²; even more preferably about 8.0 N/mm², for example 8.2 N/mm² or 8.5 N/mm².

In an embodiment the component enclosure 71 material has an elongation, defined by the maximal elongation at break as measured in accordance with ISO 37 type 1, in the range of at least 100% to at most 800%; preferably 200% to 800%; more preferably 300% to 750%; even more preferably 350% to 750%; even more preferably 400% to 700%; even more preferably 450% to 650%; even more preferably 500% to 600%; for example, 550% or 590%.

In an embodiment the component enclosure 71 material has a tear strength, as measured in accordance with ASTM D 624 BN, in the range of at least 20 to at most 30 N/mm; preferably 21 to 29 N/mm; more preferably 21 to 28 N/mm; even more preferably 22 to 27 N/mm; even more preferably 23 to 26 N/mm; for example, 24 N/mm or 25 N/mm.

In an embodiment component enclosure 71 comprises, preferably consists of, silicone rubber(SR), more preferably liquid silicone rubber (LSR); a thermoplastic elastomer (TPE), more preferably thermoplastic polyurethane (TPU), thermoplastic olefin (TPO), thermoplastic polyamide (TPA); a thermoplastic vulcanizate (TPV), a cross-linkable elastomer, more preferably isoprene rubber (IR), nitrile rubber (NBR), styrene-butadiene rubber (SBR), Ethlylene Propylene Diene Monomer (EPDM), fluorocarbon-based fluoroelastomer materials (FKM), polybutadiene rubber (BR); and/or combinations thereof.

According to alternative embodiments, the component enclosure 71 could comprise a hardness, stiffness, tensile strength, rigidity, that is higher, than the values mentioned above, such as for example an increase of 20% or more, 50% or more, 100% or more, at least 2 times higher, at least 5 times higher with respect to the materials mentioned above and/or the material of the cover 40 and/or the material of the patch 10 of the patch assembly 1. According to such embodiments, the material may comprise and or consist of a thermoplastic material, a thermohardening material, metal, ... . Similarly, the material of the component enclosure 71 could comprise a reduced elasticity and/or flexibility with respect to the material of the cover 40 and/or the patch 10. For example, a reduction of 20% or more, a reduction of 50% or more, a reduction of 100% or more, at least 2 times less, at least 5 times less, when compared to the flexibility and/or elasticity of the materials mentioned above and/or the material of the cover 40 and/or the material of the patch 10 of the patch assembly 1. Preferably the material provides for a rigidity and strength that allows for protection of the component 70 against for example sudden impact forces. Preferably the component enclosure 71 is configured to protect the component 70 against moisture ingress. According to an embodiment the component 70 comprises the component enclosure 71.

For convenience and clarity, patch 10 was discussed in the present disclosure as a monolayer consisting of a single material. Such simple embodiments notwithstanding, in practice a patch 10 may typically be multi-layered, *i*.*e*., comprising multiple layers of the same or different materials. As such, each layer may be defined by the same or different material properties depending on its purpose.

In an embodiment a most proximally positioned patch layer facing the subject's skin may be configured to adhere to the subject's skin tissue, optionally with the presence of an adhesive, such that the patch 1 can remain stuck and not release during physical movement. However, any further distally positioned patch layer may not require such skin adhesive properties and can therefore be configured for other structural purposes. For example, one or more distally positioned patch layer may be configured for a specific rigidity, flexibility or stretchability, dependent on the patch assembly application and placement on the subject's body. For example, a medical application may require different material parameters than a sports application. The skilled person can select a suitable material for its intended purpose. It is understood that these embodiments can be easily expanded or combined.

In an embodiment stretch and flex tracks may be provided in the patch, preferably provided between or across different layers of a multi-layer patch. As used herein the stretch and flex tracks refer to segments of the patch that have an increased stretchability and flexibility compared to other parts of same patch 10. This may further improve wearing comfort and/or prevent release of the patch 10 during movement.

In a preferred embodiment the stretch and flex track may provide space for a wired connection, such as an electrical lead 81 as described in the present disclosure, for connecting two or more separate electronic components. As such, the stretch and flex track are preferably arranged between an electronic component, like a sensor, and its corresponding electrodes or receiving component. An additional advantage is that this allows component 70 to be mounted away from its corresponding electrodes such that the components 70 and electrodes do not substantially overlap. This might be beneficial for the stretching and/or retracting properties of cover 40 and/or patch 10, and to prevent release of said component 70. Similar advantages may be provided for embodiments comprising an additional component 80 as discussed further below.

In an embodiment the patch 10 material has a hardness, defined by a Shore A hardness value as measured in accordance with ISO 7619-1, in the range of at least 10 shore A to at most 90 shore A; preferably 20 shore A to 80 shore A; more preferably 30 shore A to 70 shore A; even more preferably 30 shore A to 60 shore A; for example, 40 shore A or 50 shore A.

In an embodiment the patch 10 material has a tensile strength, as measured in accordance with ISO 37 type 1, in the range of at least 5.0 N/mm² to at most 11.0 N/mm²; preferably 5.5 N/mm² to at most 10.5 N/mm²; more preferably 6.0 N/mm² to at most 10.0 N/mm²; even more preferably 6.5 N/mm² to at most 9.5 N/mm²; even more preferably 7.0 N/mm² to at most 9.0 N/mm²; even more preferably 7.5 N/mm² to at most 8.5 N/mm²; even more preferably about 8.0 N/mm², for example 8.2 N/mm² or 8.5 N/mm².

In an embodiment the patch 10 material has an elongation, defined by the maximal elongation at break as measured in accordance with ISO 37 type 1, in the range of at least 100% to at most 800%; preferably 200% to 800%; more preferably 300% to 750%; even more preferably 350% to 750%; even more preferably 400% to 700%; even more preferably 450% to 650%; even more preferably 500% to 600%; for example, 550% or 590%.

In an embodiment the patch 10 material has a tear strength, as measured in accordance with ASTM D 624 BN, in the range of at least 20 to at most 30 N/mm; preferably 21 to 29 N/mm; more preferably 21 to 28 N/mm; even more preferably 22 to 27 N/mm; even more preferably 23 to 26 N/mm; for example, 24 N/mm or 25 N/mm.

In an embodiment patch 10 comprises, preferably consists of, silicone rubber(SR), more preferably liquid silicone rubber (LSR); a thermoplastic elastomer (TPE), more preferably thermoplastic polyurethane (TPU), thermoplastic olefin (TPO), thermoplastic polyamide (TPA); a thermoplastic vulcanizate (TPV), a cross-linkable elastomer, more preferably isoprene rubber (IR), nitrile rubber (NBR), styrene-butadiene rubber (SBR), Ethlylene Propylene Diene Monomer (EPDM), fluorocarbon-based fluoroelastomer materials (FKM), polybutadiene rubber (BR); and/or combinations thereof.

In an embodiment the cover 40 and by extension the patch assembly 1 may be configured to hold multiple, i.e., more than one, for example two, components 70, 80. These components may be connected such that they can interact with each other. In such an embodiment a wired connection such as an electrical lead 81 may be provided connecting the at least two components 70,80. Alternatively, the components may be configured to interact wirelessly, for example via Bluetooth, such that no wired connection is required. It is understood that the cover 40 can be easily adapted to hold more than two components, such as three or four components, but for brevity only an embodiment comprising two components 70,80 will be discussed below.

With reference to **Figure 14****,** an embodiment of patch assembly 1 is shown wherein a second component 80 is disposed between patch 10 and a further layer 11 of said patch. It is further shown that component 80 is electrically connected to component 70 with an electrical lead 81 and contains 2 electrodes arranged on the proximal side of the patch facing the subject's skin. For example, component 80 may be a sensor configured to sense a physiological status of the subject and optionally, after sensing, transmit the raw or processed sensor data via the electrical lead 81 to component 70 for further processing. Advantageously, stretch and flex tracks may be provided between the electronics and electrodes.

With reference to **Figure 13****,** it is shown that component 80 is integrated into patch 10; it forms a non-removable component of the patch 10. Such an embodiment may be suitable for a disposable patch 10, in particular wherein patch 10 and component 80 are disposed after use, whereas component 70 can be removed and transferred into an unused patch, optionally provided with another component 80, for further use.

With reference to **Figure 18****,** another embodiment of patch assembly 1 is shown wherein a second component 80 is disposed in a separate cover assembly in a manner similar to component 70. It is shown that component 80 is also a removable component of patch assembly 1. In the present embodiment both component 70 and component 80 may be removed or replaced from the patch 10 for further use. As is further shown, component 70 can interact with component 80 via an electrical lead 81 embedded in patch 10, for example to exchange data.

It is further clear that, according to some preferred embodiments, similar as the embodiments described above the cover 40 is configured as a separate and/or different element from the patch 10. It is thus clear that, although the cover 40 and the patch 10 each could be embodied as single layer or multi-layer components, that both the cover 40 and the patch 10 are structurally and functionally separate and different elements. The patch 10 of the patch assembly 1 is configured to provide adherence to skin of a subject. The cover 40 of the patch assembly 1 is configured to at least partially enclose the component 70. Preferably the cover 40 is not configured for attachment to skin of the subject to which the patch 10 is attached. In other words, according to such embodiments, when in use, the patch assembly 1 comprises a patch adhered to the skin of a subject and a cover 40, which is a different or separate element from the patch 10, the cover 40 at least partially enclosing the component 70, the cover 40 not being attached to skin of the subject, this means, not being directly attached, adhered and/or in touch with the skin of the subject.

Preferably, similar as shown in the embodiments above, only the cover 40 is configured to clamp the component 70 when mounted inside the cover 40. In other words, according to such embodiments, there is provided a patch assembly 1 in which only the cover 40 clamps the component 70. It is clear that such embodiments, preferably only the cover 40 is configured to apply the compression force to the component 70. In other words, according to such embodiments, there is provided a patch assembly 1 in which the only the cover 40 applies the compression force to the component 70, thereby clamping the component 70 at least partly enclosed by the cover 40. This thus means that according to such preferred embodiments the patch assembly does not comprise any other elements which function to clamp the component by means of applying a compression force to the component 70, thereby attaching the component 70 to the inside of the cover 40. More particularly, according to such embodiments the patch 10 does not clamp or cooperate with the cover 40 to clamp the component 40 inside the cover 40. Similarly, according to such embodiments of the patch assembly 1 the patch 10 does not apply a compression force or cooperates with the cover 40 to apply a compression force to the component 70. is configured not to clamp the component 70 when mounted inside the cover 40. In other words, according to such embodiments the patch 10 is configured not to apply the compression force to the component 70 when mounted inside the cover 40.

Preferably, similar as in the embodiments shown above, patch 10 is configured not to contact the component 70 when mounted inside the cover 40. In other words, the patch assembly 1 comprises a patch 10 which does not contact the component 70 when mounted inside the cover 40. Similar to the embodiments described above, the cover 40 for example comprises a proximal or an inferior portion 45 configured to at least partially cover a proximal or an inferior side of component 70, or in other words the side of the component 70 which closest to the patch 10 of the patch assembly 1. It is thus clear that, similar as described above, according to particular embodiments, in this way, the inferior portion 45 of the cover 40 defines a gap between the component 70 and the patch 10. Preferably the inferior portion 45 of the cover 40 ensures that there is created a space or passage for easier passage of gas or fluids for breathing of the skin or ensures that the component 70 does not contact the patch 10 or skin. Similar as for the embodiments described above, preferably, the cover 40 comprises at least one pair of clamping parts 41 arranged at opposing sides of the component 70, whereby, each of such a pair of clamping parts 41 comprises the inferior portion 45 of the cover 40 or a part thereof, which is arranged between the component 70 and the patch 10.

It is further clear that, according to the embodiments of the patch assembly 1, such as for example shown in the drawings the cover 40 comprises at least one pair of clamping parts 41 at opposing sides of the component 70. It is further clear that according to such embodiments, each of such a pair of clamping parts 41 comprises preferably an inferior portion 45, which is arranged between the component 70 and the patch 10. It is further clear that according to such embodiments, wherein such a pair of clamping parts 40 is arranged at opposing sides of the component 70, more specifically, for example a pair of clamping parts 41 of the cover 40 at opposing sides of the component 70 along the lateral direction L. In other words, at opposing sides of the component 70 along a direction parallel or substantially parallel with the planar patch 10 of the patch assembly 1. In other words, in the orientation shown in the Figures, a pair of clamping parts 41 positioned sideways, or in other words to the left and the right at opposing sides of the component 70. It is clear that according to such embodiments the cover 40 could comprise two such pairs of clamping pars 41 at opposing sides of the component 70, such as for example, for a component 70, with a rectangular or substantially rectangular shape, when seen from the top view, a first pair of clamping parts 41 at a first pair of opposing sides of the circumference of this rectangular shape, and a second pair of clamping parts 41 at the other pair of opposing sides of the circumference of this rectangular shape. It is clear that according to such embodiments, the cover 40 comprises at least two flexible and stretchable clamping parts 41 at opposing sides of the component 70 when mounted, wherein the at least two flexible and stretchable clamping parts 41 are arranged in such a way that the at least two flexible and stretchable clamping parts 41 cooperate to apply the compression force to attach the component 70 to the inside of the cover 40 between the at least two flexible and stretchable clamping parts 41. It is clear that still further alternative embodiments are possible in which the cover 40 comprises at least two flexible and stretchable clamping parts 41, preferably arranged at opposing sides of the component 70 when mounted. Similar according to the embodiments described above, it is clear that still furter embodiments are possible in which the cover 40 comprises at least two flexible and stretchable clamping parts 41 configured to apply the compression force to the component 70 when mounted in between the at least two clamping parts 41.

Preferably, as illustrated by means of the embodiments above, the cover 40, and more particularly the clamping parts 41 of the cover, consists of a single-piece element. Although, such embodiment of a cover, could for example be embodied as a single layer, or multi-layer element, it is clear that such a cover 40, and more particular the clamping parts 41 thereof, are not composed of different elements of the patch assembly 1 or parts thereof. In other words, it is clear that the cover 40 partially encloses the component 40, preferably with a pair of clamping parts 41 at opposing sides of the component, as a single-piece element. According to such embodiments the cover, and more particularly, the clamping parts 41, do not comprise any other elements, or parts thereof, such as for example the patch 10 or parts thereof, or any other element of the patch assembly 10, to enable the cover to partially enclose and clamp the component inside the cover 40 by applying a compression force to the component 70. According to such embodiments, thus preferably only the cover 40, and more particularly only the clamping parts 41 of the cover exhibit a compression force to the component to attach the component to the inside of the cover 40.

It is further clear that in the context of this description, to clamp, or clamping is to be interpreted as to fasten or hold with a clamp, wherein a clamp is a device comprising preferably at least two clamping parts configured to exert a compression force on something when two clamping parts are urged closer together.

## Claims

1. A patch assembly (1) configured for attachment to a subject's skin comprising:
- a patch (10) configured for removeable attachment to the skin of said subject, and
- at least one cover assembly configured for attachment to the patch (10), and the at least one cover assembly respectively comprising:
- a flexible and stretchable cover (40) configured for attachment to the patch (10) and at least partially enclosing a component (70), whereby the cover (40) consists of a single-piece element and the cover (40) comprises at least two flexible and stretchable clamping parts (41) arranged at opposing sides of the component (70) when mounted configured :
- to clamp the component (70) when mounted inside the cover (40) in such a way that the clamping parts (41) are in contact with the component (70); and
- to apply a compression force to the component (70) when mounted in between the at least two clamping parts (41), thereby attaching the component (70) to the inside of the cover (40); and
- the component (70) mounted inside the cover (40) by means of the at least two flexible and stretchable clamping parts (41),
whereby the at least one cover assembly is attached to the patch (10) by means of the cover (40) of said cover assembly.

2. The patch assembly according to claim 1, wherein the clamping parts (41) are configured to clamp the component (70) when mounted inside the cover (40) in such a way that the clamping parts (41) are in slidable contact with the component (70).

3. The patch assembly according to any one of the preceding claims, wherein:
- the cover (40) is configured for attachment at one or more attachment points (44) to a patch (10); and
- the patch (10) is configured for attachment to skin of a subject;
- and optionally, the cover (40) is configured as a separate and/or different element from the patch (10);
- and optionally, the cover (40) is not configured for attachment to skin of the subject;
- and optionally, only the cover (40) is configured to clamp the component (70) when mounted inside the cover (40);
- and optionally, only the cover (40) is configured to apply the compression force to the component (70);
- and optionally, the patch (10) is configured not to clamp the component (70) when mounted inside the cover (40);
- and optionally, the patch (10) is configured not to apply the compression force to the component (70) when mounted inside the cover (40);
- and optionally, the patch (10) is configured not to contact the component (70) when mounted inside the cover (40).

4. The patch assembly according to any one of the preceding claims, wherein:
- the at least two clamping parts (41) are configured to apply the compression force to the component (70), according to at least one of the following options:
- along at least one direction;
- along a plurality of directions;
- inwards along at least a part of an outer surface of the component (70);
- and optionally, wherein the cover (40) comprises at least two flexible and stretchable clamping parts (41) at opposing sides of the component (70) when mounted, wherein the at least two flexible and stretchable clamping parts (41) are arranged in such a way that the at least two flexible and stretchable clamping parts (41) cooperate to apply the compression force to attach the component (70) to the inside of the cover (40) between the at least two flexible and stretchable clamping parts (41).

5. The patch assembly according to any one of the preceding claims, wherein the cover consists of a material defined by at least one of the following parameters:
- a hardness in the range of at least 10 shore A to at most 90 shore A; preferably 20 shore A to 80 shore A; more preferably 30 shore A to 70 shore A; even more preferably 30 shore A to 60 shore A;
- a tensile strength in the range of at least 5.0 N/mm² to at most 11.0 N/mm²; preferably 5.5 N/mm² to at most 10.5 N/mm²; more preferably 6.0 N/mm² to at most 10.0 N/mm²; even more preferably 6.5 N/mm² to at most 9.5 N/mm²; even more preferably 7.0 N/mm² to at most 9.0 N/mm²; even more preferably 7.5 N/mm² to at most 8.5 N/mm²;
- a maximal elongation in the range of at least 100% to at most 800%; preferably 200% to 800%; more preferably 300% to 750%; even more preferably 350% to 750%; even more preferably 400% to 700%; even more preferably 450% to 650%; even more preferably 500% to 600%; and/or
- a tear strength in the range of at least 20 to at most 30 N/mm; preferably 21 to 29 N/mm; more preferably 21 to 28 N/mm; even more preferably 22 to 27 N/mm; even more preferably 23 to 26 N/mm.

6. The patch assembly according to any one of the preceding claims, wherein the cover material comprises, preferably consists of, silicone rubber (SR), more preferably liquid silicone rubber (LSR); a thermoplastic elastomer (TPE), more preferably thermoplastic polyurethane (TPU), thermoplastic olefin (TPO), thermoplastic polyamide (TPA); a thermoplastic vulcanizate (TPV), a cross-linkable elastomer, more preferably isoprene rubber (IR), nitrile rubber (NBR), styrene-butadiene rubber (SBR), Ethlylene Propylene Diene Monomer (EPDM), fluorocarbon-based fluoroelastomer materials (FKM), polybutadiene rubber (BR); and/or combinations thereof.

7. The patch assembly according to any one of the preceding claims, wherein the cover (40), preferably the at least two flexible and stretchable clamping part (41):
- are in direct contact with the component (70) over at least part of its interior surface; and/or
- are provided with one or more inward projections (47) that are in direct contact with the component (70), wherein optionally the inward projections (47) are dimensioned to increase stretchability and/or flexibility at the location of direct contact with the clamping parts (41).

8. The path assembly according to any one of the preceding claims, wherein the at least two flexible and stretchable clamping parts (41) are configured such that, when subjected to changes resulting from flexing and/or stretching of the cover (40), the material of the clamping parts (41) in contact with the component (70) is slidable with respect to the component (70).

9. The patch assembly according to any of preceding claims, wherein the component (70) comprises and/or consists of one or more of the following:
- an electronic component;
- a non-electronic component;
- a reservoir;
- a dispenser.

10. A patch assembly according to any of the preceding claims, wherein the cover (40) comprises a proximal or an inferior portion (45) configured to at least partially cover the side of the component (70) which closest to the patch (10) of the patch assembly (1), and wherein the inferior portion (45) of the cover (40) defines a gap between the component (70) and the patch (10),
and wherein preferably, the cover (40) comprises at least one pair of clamping parts (41) arranged at opposing sides of the component (70), whereby, each of such a pair of clamping parts (41) comprises the inferior portion (45) or a part thereof, which is arranged between the component (70) and the patch (10).

11. A patch assembly according to any one of the preceding claims, wherein the patch assembly further comprises:
- a sensor configured for measurement of a physiological status in electronic communication with the component (70), wherein the component (70) comprises at least one electronic circuit configured to process, store and or communicate the measurement of the sensor.

12. The patch assembly according to any one of the preceding claims, comprising a membrane (50) which is permeable to gas and preferably impermeable to fluid.

13. Use of a patch assembly according to any one of the preceding claims for measurement of a physiological status of a subject.

## Patentansprüche

1. Patch-Anordnung (1), die zur Befestigung an der Haut eines Subjekts ausgelegt ist, umfassend:
- ein Patch (10), das zur entfernbaren Befestigung an der Haut des Subjekts ausgelegt ist, und
- mindestens eine Abdeckungsanordnung, die zur Befestigung an dem Patch (10) ausgelegt ist, und wobei die mindestens eine Abdeckungsanordnung jeweils Folgendes umfasst:
- eine flexible und dehnbare Abdeckung (40), die zur Befestigung an dem Patch (10) ausgelegt ist und eine Komponente (70) mindestens teilweise umschließt, wobei die Abdeckung (40) aus einem einstückigen Element besteht und die Abdeckung (40) mindestens zwei flexible und dehnbare Klemmteile (41) umfasst, die an gegenüberliegenden Seiten der Komponente (70) angeordnet sind und, wenn sie montiert sind, ausgelegt sind, um:
- die Komponente (70), wenn sie innerhalb der Abdeckung (40) montiert ist, so zu klemmen, dass die Klemmteile (41) mit der Komponente (70) in Kontakt stehen; und
- eine Druckkraft auf die Komponente (70) auszuüben, wenn sie zwischen den mindestens zwei Klemmteilen (41) montiert ist, wodurch die Komponente (70) an der Innenseite der Abdeckung (40) befestigt ist; und
- die Komponente (70), die mittels der mindestens zwei flexiblen und dehnbaren Klemmteile (41) innerhalb der Abdeckung (40) montiert ist,
wobei die mindestens eine Abdeckungsanordnung mittels der Abdeckung (40) der Abdeckungsanordnung an dem Patch (10) befestigt ist.

2. Patch-Anordnung nach Anspruch 1, wobei die Klemmteile (41) dazu ausgelegt sind, die Komponente (70), wenn sie innerhalb der Abdeckung (40) montiert ist, derart zu klemmen, dass die Klemmteile (41) in verschiebbarem Kontakt mit der Komponente (70) sind.

3. Patch-Anordnung nach einem der vorhergehenden Ansprüche, wobei:
- die Abdeckung (40) zur Befestigung an einem oder mehreren Befestigungspunkten (44) an einem Patch (10) ausgelegt ist; und
- das Patch (10) zur Befestigung an der Haut eines Subjekts ausgelegt ist;
- und gegebenenfalls die Abdeckung (40) als ein separates und/oder verschiedenes Element von dem Patch (10) ausgelegt ist;
- und gegebenenfalls die Abdeckung (40) nicht zur Befestigung an der Haut des Subjekts ausgelegt ist;
- und gegebenenfalls nur die Abdeckung (40) dazu ausgelegt ist, die Komponente (70) zu klemmen, wenn sie innerhalb der Abdeckung (40) montiert ist;
- und gegebenenfalls nur die Abdeckung (40) dazu ausgelegt ist, die Druckkraft auf die Komponente (70) auszuüben;
- und gegebenenfalls das Patch (10) dazu ausgelegt ist, die Komponente (70) nicht zu klemmen, wenn sie innerhalb der Abdeckung (40) montiert ist;
- und gegebenenfalls das Patch (10) dazu ausgelegt ist, die Druckkraft nicht auf die Komponente (70) auszuüben, wenn sie innerhalb der Abdeckung (40) montiert ist;
- und gegebenenfalls das Patch (10) dazu ausgelegt ist, die Komponente (70) nicht zu kontaktieren, wenn sie innerhalb der Abdeckung (40) montiert ist.

4. Patch-Anordnung nach einem der vorhergehenden Ansprüche, wobei:
- die mindestens zwei Klemmteile (41) dazu ausgelegt sind, die Druckkraft auf die Komponente (70) gemäß mindestens einer der folgenden Optionen auszuüben:
- entlang mindestens einer Richtung;
- entlang mehrerer Richtungen;
- nach innen entlang mindestens eines Teils einer Außenfläche der Komponente (70);
- und wobei gegebenenfalls die Abdeckung (40) mindestens zwei flexible und dehnbare Klemmteile (41) an gegenüberliegenden Seiten der Komponente (70) umfasst, wenn sie montiert ist, wobei die mindestens zwei flexiblen und dehnbaren Klemmteile (41) derart angeordnet sind, dass die mindestens zwei flexiblen und dehnbaren Klemmteile (41) zusammenwirken, um die Druckkraft auszuüben, um die Komponente (70) an der Innenseite der Abdeckung (40) zwischen den mindestens zwei flexiblen und dehnbaren Klemmteilen (41) zu befestigen.

5. Patch-Anordnung nach einem der vorhergehenden Ansprüche, wobei die Abdeckung aus einem Material besteht, das durch mindestens einen der folgenden Parameter definiert ist:
- eine Härte im Bereich von mindestens 10 Shore A bis höchstens 90 Shore A; vorzugsweise von 20 Shore A bis 80 Shore A; bevorzugter von 30 Shore A bis 70 Shore A; noch bevorzugter von 30 Shore A bis 60 Shore A;
- eine Zugfestigkeit im Bereich von mindestens 5,0 N/mm² bis höchstens 11,0 N/mm²; vorzugsweise von 5,5 N/mm² bis höchstens 10,5 N/mm²; bevorzugter von 6,0 N/mm² bis höchstens 10,0 N/mm²; noch bevorzugter von 6,5 N/mm² bis höchstens 9,5 N/mm²; noch bevorzugter von 7,0 N/mm² bis höchstens 9,0 N/mm²; noch bevorzugter von 7,5 N/mm² bis höchstens 8,5 N/mm²;
- eine maximale Dehnung im Bereich von mindestens 100 % bis höchstens 800 %; bevorzugt von 200 % bis 800 %; besonders bevorzugt von 300 % bis 750 %; bevorzugter von 350 % bis 750 %; noch bevorzugter von 400 % bis 700 %; noch bevorzugter von 450 % bis 650 %; noch bevorzugter von 500 % bis 600 %; und/oder
- eine Reißfestigkeit im Bereich von mindestens 20 bis höchstens 30 N/mm; vorzugsweise von 21 bis 29 N/mm; bevorzugter von 21 bis 28 N/mm; noch bevorzugter von 22 bis 27 N/mm; noch bevorzugter von 23 bis 26 N/mm.

6. Patch-Anordnung nach einem der vorhergehenden Ansprüche, wobei das Abdeckungsmaterial Silikonkautschuk (SR), bevorzugter flüssigen Silikonkautschuk (LSR); ein thermoplastisches Elastomer (TPE), bevorzugter thermoplastisches Polyurethan (TPU), thermoplastisches Olefin (TPO), thermoplastisches Polyamid (TPA); ein thermoplastisches Vulkanisat (TPV), ein vernetzbares Elastomer, bevorzugter Isoprenkautschuk (IR), Nitrilkautschuk (NBR), Styrolbutadienkautschuk (SBR), Ethlylenpropylendien-Monomer (EPDM), Fluorkohlenwasserstoff-basierte Fluor-Elastomermaterialien (FKM), Polybutadienkautschuk (BR); und/oder Kombinationen davon umfasst oder vorzugsweise daraus besteht.

7. Patch-Anordnung nach einem der vorhergehenden Ansprüche, wobei die Abdeckung (40), vorzugsweise die mindestens zwei flexiblen und dehnbaren Klemmteile (41):
- über mindestens einen Teil ihrer Innenfläche in direktem Kontakt mit der Komponente (70) stehen; und/oder
- mit einem oder mehreren nach innen gerichteten Vorsprüngen (47) versehen sind, die in direktem Kontakt mit der Komponente (70) stehen, wobei gegebenenfalls die nach innen gerichteten Vorsprünge (47) so dimensioniert sind, dass sie die Dehnbarkeit und/oder Flexibilität an der Stelle des direkten Kontakts mit den Klemmteilen (41) erhöhen.

8. Patch-Anordnung nach einem der vorhergehenden Ansprüche, wobei die mindestens zwei flexiblen und dehnbaren Klemmteile (41) so ausgelegt sind, dass, wenn sie Änderungen unterworfen werden, die durch Biegen und/oder Dehnen der Abdeckung (40) resultieren, das Material der Klemmteile (41), die mit der Komponente (70) in Kontakt stehen, in Bezug auf die Komponente (70) verschiebbar ist.

9. Patch-Anordnung nach einem der vorhergehenden Ansprüche, wobei die Komponente (70) eines oder mehrere des Folgenden umfasst und/oder daraus besteht:
- eine elektronische Komponente;
- eine nichtelektronische Komponente;
- einen Behälter;
- einen Spender.

10. Patch-Anordnung nach einem der vorhergehenden Ansprüche, wobei die Abdeckung (40) einen proximalen oder einen unteren Abschnitt (45) umfasst, der dazu ausgelegt ist, die Seite der Komponente (70), die dem Patch (10) der Patch-Anordnung (1) am nächsten liegt, mindestens teilweise abzudecken, und wobei der untere Abschnitt (45) der Abdeckung (40) einen Spalt zwischen der Komponente (70) und dem Patch (10) definiert,
und wobei vorzugsweise die Abdeckung (40) mindestens ein Paar Klemmteile (41) umfasst, die an gegenüberliegenden Seiten der Komponente (70) angeordnet sind, wobei jedes derartige Paar Klemmteile (41) den unteren Abschnitt (45) oder einen Teil davon umfasst, der zwischen der Komponente (70) und dem Patch (10) angeordnet ist.

11. Patch-Anordnung nach einem der vorhergehenden Ansprüche, wobei die Patch-Anordnung ferner Folgendes umfasst:
- einen Sensor, der zum Messen eines physiologischen Status in elektronischer Kommunikation mit der Komponente (70) ausgelegt ist, wobei die Komponente (70) mindestens eine elektronische Schaltung umfasst, die zum Verarbeiten, Speichern und/oder Kommunizieren der Messung des Sensors ausgelegt ist.

12. Patch-Anordnung nach einem der vorhergehenden Ansprüche, umfassend eine Membran (50), die gasdurchlässig und vorzugsweise fluidundurchlässig ist.

13. Verwendung einer Patch-Anordnung nach einem der vorhergehenden Ansprüche zur Messung eines physiologischen Status eines Subjekts.

## Revendications

1. Ensemble timbre (1) configuré pour fixation sur la peau d'un sujet comprenant :
- un timbre (10) configuré pour fixation amovible sur la peau dudit sujet, et
- au moins un ensemble couvercle configuré pour fixation sur le timbre (10), et l'au moins un ensemble couvercle comprenant respectivement :
- un couvercle (40) flexible et extensible configuré pour fixation sur le timbre (10) et renfermant au moins partiellement un composant (70), moyennant quoi le couvercle (40) est constitué d'un élément en une seule pièce et le couvercle (40) comprend au moins deux parties de serrage (41) flexibles et extensibles agencées au niveau de côtés opposés du composant (70) lorsque monté configuré :
- pour serrer le composant (70) lorsque monté à l'intérieur du couvercle (40) de sorte que les parties de serrage (41) soient en contact avec le composant (70) ; et
- pour appliquer une force de compression sur le composant (70) lorsque monté entre les au moins deux parties de serrage (41), fixant ainsi le composant (70) sur l'intérieur du couvercle (40) ; et
- le composant (70) monté à l'intérieur du couvercle (40) au moyen des au moins deux parties de serrage (41) flexibles et extensibles,
moyennant quoi l'au moins un ensemble couvercle est fixé sur le timbre (10) au moyen du couvercle (40) dudit ensemble couvercle.

2. Ensemble timbre selon la revendication 1, dans lequel les parties de serrage (41) sont configurées pour serrer le composant (70) lorsque monté à l'intérieur du couvercle (40) de sorte que les parties de serrage (41) soient en contact coulissant avec le composant (70).

3. Ensemble timbre selon l'une quelconque des revendications précédentes, dans lequel :
- le couvercle (40) est configuré pour fixation sur un ou plusieurs points de fixation (44) sur un timbre (10) ; et
- le timbre (10) est configuré pour fixation sur la peau d'un sujet ;
- et facultativement, le couvercle (40) est configuré comme un élément séparé et/ou différent du timbre (10) ;
- et facultativement, le couvercle (40) n'est pas configuré pour fixation sur la peau du sujet ;
- et facultativement, seul le couvercle (40) est configuré pour serrer le composant (70) lorsque monté à l'intérieur du couvercle (40) ;
- et facultativement, seul le couvercle (40) est configuré pour appliquer la force de compression sur le composant (70) ;
- et facultativement, le timbre (10) est configuré pour ne pas serrer le composant (70) lorsque monté à l'intérieur du couvercle (40) ;
- et facultativement, le timbre (10) est configuré pour ne pas appliquer la force de compression sur le composant (70) lorsque monté à l'intérieur du couvercle (40) ;
- et facultativement, le timbre (10) est configuré pour ne pas être en contact avec le composant (70) lorsque monté à l'intérieur du couvercle (40).

4. Ensemble timbre selon l'une quelconque des revendications précédentes, dans lequel :
- les au moins deux parties de serrage (41) sont configurées pour appliquer la force de compression sur le composant (70), selon au moins l'une des options suivantes :
- le long au moins d'une direction ;
- le long d'une pluralité de directions ;
- vers l'intérieur le long au moins d'une partie d'une surface externe du composant (70) ;
- et facultativement, dans lequel le couvercle (40) comprend au moins deux parties de serrage (41) flexibles et extensibles au niveau de côtés opposés du composant (70) lorsque monté, les au moins deux parties de serrage (41) flexibles et extensibles étant agencées de sorte que les au moins deux parties de serrage (41) flexibles et extensibles coopèrent pour appliquer la force de compression afin de fixer le composant (70) sur l'intérieur du couvercle (40) entre les au moins deux parties de serrage (41) flexibles et extensibles.

5. Ensemble timbre selon l'une quelconque des revendications précédentes, dans lequel le couvercle est constitué d'un matériau défini par au moins un des paramètres suivants :
- une dureté dans la plage d'au moins 10 Shore A à 90 Shore A au plus ; préférablement 20 Shore A à 80 Shore A ; plus préférablement 30 Shore A à 70 Shore A ; encore plus préférablement 30 Shore A à 60 Shore A ;
- une résistance à la traction dans la plage d'au moins 5,0 N/mm² à 11,0 N/mm² au plus ; préférablement 5,5 N/mm² à 10,5 N/mm² au plus ; plus préférablement 6,0 N/mm² à 10,0 N/mm² au plus ; encore plus préférablement 6,5 N/mm² à 9,5 N/mm² au plus ; encore plus préférablement 7,0 N/mm² à 9,0 N/mm² au plus ; encore plus préférablement 7,5 N/mm² à 8,5 N/mm² au plus ;
- un allongement maximal dans la plage d'au moins 100 % à 800 % au plus ; préférablement 200 % à 800 % ; plus préférablement 300 % à 750 % ; encore plus préférablement 350 % à 750 % ; encore plus préférablement 400 % à 700 % ; encore plus préférablement 450 % à 650 % ; encore plus préférablement 500 % à 600 % ; et/ou
- une résistance au déchirement dans la plage d'au moins 20 à 30 N/mm au plus ; préférablement 21 à 29 N/mm ; plus préférablement 21 à 28 N/mm ; encore plus préférablement 22 à 27 N/mm ; encore plus préférablement 23 à 26 N/mm.

6. Ensemble timbre selon l'une quelconque des revendications précédentes, dans lequel le matériau de couvercle comprend, préférablement est constitué de, caoutchouc silicone (SR), plus préférablement caoutchouc silicone liquide (LSR) ; un élastomère thermoplastique (TPE), plus préférablement polyuréthane thermoplastique (TPU), oléfine thermoplastique (TPO), polyamide thermoplastique (TPA) ; un vulcanisat thermoplastique (TPV), un élastomère réticulable, plus préférablement caoutchouc isoprène (IR), nitrile de caoutchouc (NBR), caoutchouc styrène-butadiène (SBR), monomère de diène de propylène d'éthylène (EPDM), matériaux de fluoroélastomère à base de fluorocarbone (FKM), caoutchouc de polybutadiène (BR) ; et/ou leurs combinaisons.

7. Ensemble timbre selon l'une quelconque des revendications précédentes, dans lequel le couvercle (40), préférablement les au moins deux parties de serrage (41) flexibles et extensibles :
- sont en contact direct avec le composant (70) sur au moins une partie de sa surface intérieure ; et/ou
- sont pourvues d'une ou de plusieurs saillies vers l'intérieur (47) qui sont en contact direct avec le composant (70), les saillies vers l'intérieur (47) étant facultativement dimensionnées pour augmenter l'extensibilité et/ou la flexibilité au niveau de l'endroit de contact direct avec les parties de serrage (41).

8. Ensemble timbre selon l'une quelconque des revendications précédentes, dans lequel les au moins deux parties de serrage (41) flexibles et extensibles sont configurées de telle sorte que, lorsque soumises à des changements résultant d'une flexion et/ou d'une extension du couvercle (40), le matériau des parties de serrage (41) en contact avec le composant (70) peut coulisser par rapport au composant (70).

9. Ensemble timbre selon l'une quelconque des revendications précédentes, dans lequel le composant (70) comprend et/ou est constitué d'un ou de plusieurs des éléments suivants :
- un composant électronique ;
- un composant non-électronique ;
- un réservoir ;
- un distributeur.

10. Ensemble timbre selon l'une quelconque des revendications précédentes, dans lequel le couvercle (40) comprend une portion proximale ou une portion inférieure (45) configurée pour couvrir au moins partiellement le côté du composant (70) qui est le plus proche du timbre (10) de l'ensemble timbre (1), et la portion inférieure (45) du couvercle (40) définissant un intervalle entre le composant (70) et le timbre (10),
et dans lequel préférablement, le couvercle (40) comprend au moins une paire de pièces de serrage (41) agencées au niveau de côtés opposés du composant (70), moyennant quoi, chaque telle paire de pièces de serrage (41) comprend la portion inférieure (45) ou une partie de celle-ci, qui est agencée entre le composant (70) et le timbre (10).

11. Ensemble timbre selon l'une quelconque des revendications précédentes, l'ensemble timbre comprenant en outre :
- un capteur configuré pour mesure d'un état physiologique en communication électronique avec le composant (70), le composant (70) comprenant au moins un circuit électronique configuré pour traiter, stocker et ou communiquer la mesure du capteur.

12. Ensemble timbre selon l'une quelconque des revendications précédentes, comprenant une membrane (50) qui est perméable aux gaz et préférablement imperméable aux fluides.

13. Utilisation d'un ensemble timbre selon l'une quelconque des revendications précédentes pour mesure d'un état physiologique d'un sujet.
